# EUROPEAN PATENT APPLICATION

(11) **EP 3 020 819 A1**
(43) Date of publication of application: **18.05.2016**
(21) Application number: 14193474.5
(22) Date of filing: 17.11.2014
(51) Int. Cl.: C12P 7/64

(54) **Fatty acid and derivatives production**

(71) Applicant: Evonik Degussa GmbH, 45128 Essen (DE)
(72) Inventor: Haas, Thomas, 48161 Münster (DE); Pötter, Markus, 201702 Shanghai (CN); Schaffer, Steffen, 45699 Herten (DE)

(57) **Abstract**

The present invention relates to at least one method of producing at least one fatty acid and/or derivative thereof from a gas comprising H₂, CO₂ and O₂ the method comprising the steps of:
(c) providing a genetically modified hydrogen oxidising bacterium in an aqueous medium; and
(d) contacting the aqueous medium with a gas comprising H₂, CO₂ and O₂ in a weight ratio of 20 to 70: 10 to 45: 5 to 35;

wherein the fatty acid comprises at least 5 carbon atoms and
wherein the hydrogen oxidising bacterium is genetically modified relative to the wild type bacterium to increase the expression of enzyme E₁ that is capable of catalysing the conversion of acetyl CoA to acyl ACP via malonyl coA and to increase the expression of enzyme E₂ that is capable of catalysing the conversion of Acyl ACP to the fatty acid.

## Description

### FIELD OF THE INVENTION

The present invention relates to fatty acid production by biotechnological means. In particular, the present invention relates to the biotechnological production of fatty acids with more than 5 carbon atoms from a renewable source.

### BACKGROUND OF THE INVENTION

Fatty acids and/or triacylglycerides have various uses in a myriad of industries. In the food industry for example, fatty acids and/or triacylglycerides can be used in animal feed and to supplement nutrition. Fatty acids and/or triacylglycerides can also be used in the cosmetic and pharmacological field. These applications may either require free fatty acids or triacylglycerides. The natural source of these fatty acids and/or triacylglycerides is rather limited.

For example, various polyunsaturated fatty acids (PUFA) and PUFA-containing triglycerides are mainly obtained from microorganisms such as *Mortierella* and *Schizochytrium* or from oil-producing plants such as soybean or oilseed rape, algae such as *Crypthecodiniumor Phaeodactylum* and others, where they are usually obtained in the form of their triacylglycerides. The free PUFA are usually prepared from the triacylglycerides by hydrolysis. However, long chain polyunsaturated fatty acids cannot be efficiently isolated from natural oil crop plants.

Traditionally, fatty acids and/or triacylglycerides produced at an industrial scale start with materials derived from petrochemicals. This usually starts with cracking gasoline or petroleum which is bad for the environment. Also, since the costs for these starting materials will be linked to the price of petroleum, with the expected increase in petroleum prices in the future, prices of fatty acids and/or triacylglycerides may also increase relative to the increase in the petroleum prices.

Accordingly, it is desirable to find more sustainable raw materials, other than purely petroleum based, as starting materials for fatty acids and/or triacylglycerides production which also cause less damage to the environment.

A range of biotechnological routes towards production of fatty acids has been described in the past. However, none of them is adequate for the commercial large-scale production of fatty acids from a renewable energy source owing to low yields, insufficient purities and the need for multi-step purification procedures. In particular, only a small proportion of the carbon substrates fed to biotechnologically useful organisms is actually converted to the sought-after product, whilst much of it is consumed by reactions of the primary metabolism.

Another problem associated with biotechnological routes is the fact that a mixture of products is obtained and thus the composition is difficult to control. More specifically, a range of fatty acids may be produced, even though production of a single adduct may be desirable. Since the mixture comprises compounds highly related in terms of chemical structure, purifying or at least enriching a single component in an efficient and straightforward manner is usually beyond technical feasibility.

Accordingly, there is a need in the art for a more efficient means of production of fatty acids from a renewable energy source.

### DESCRIPTION OF THE INVENTION

The method according to any aspect of the present invention attempts to solve the problems mentioned above. In particular, the method according to any aspect of the present invention may be able to use at least one genetically modified hydrogen oxidising bacteria to convert a carbon source to at least one fatty acid and/or derivative thereof by way of first converting the carbon source to acyl CoA and then converting the acyl CoA to malonyl coA and then finally converting the malonyl coA to at least one fatty acid with more than 5 carbon atoms. This method according to any aspect of the present invention allows for the production of fatty acid and derivatives thereof in the absence of energy-rich organic compounds. Also, the method according to any aspect of the present invention provides an efficient biotechnological route towards the production of fatty acid and derivatives thereof. In the method according to any aspect of the present invention, more carbon atoms in the fatty acid and derivatives thereof obtained are derived from carbon dioxide molecules, i.e. the proportion of carbon atoms derived from carbon dioxide rather than from any other organic molecules, for example alcohols or carbohydrates, is higher using the method according to any aspect of the present invention compared to other methods known in the art.

According to one aspect of the present invention, there is provided a method of producing at least one fatty acid and/or derivative thereof from a gas comprising H₂, CO₂ and/or O₂ the method comprising the steps of:
(a) providing a genetically modified hydrogen oxidising bacterium in an aqueous medium; and
(b) contacting the aqueous medium with a gas comprising H₂, CO₂ and/or O₂ in a weight ratio of 20 to 70: 10 to 45: 5 to 35;
wherein the fatty acid comprises at least 5 carbon atoms and
wherein the hydrogen oxidising bacterium is genetically modified relative to the wild type bacterium to increase the expression of enzyme E₁ that is capable of catalysing the conversion of acetyl CoA to acyl ACP via malonyl coA and to increase the expression of enzyme E₂ that is capable of catalysing the conversion of Acyl ACP to the fatty acid.
The genetically modified hydrogen oxidising bacterium according to any aspect of the present invention refers to recombinant hydrogen oxidising that may be engineered to yield various types of fatty acid and derivatives thereof including, but not limited to, short chain alcohols such as ethanol, propanol isopropanol and butanol, fatty alcohols, fatty acid esters, hydrocarbons, wax esters and the like.

In one example, the disclosure provides a method for modifying a hydrogen oxidising bacterium so that it produces, and optionally releases, fatty acids and/or derivatives thereof generated from a renewable carbon source. Such hydrogen oxidising bacteria are genetically engineered, for example, by introducing an exogenous DNA sequence encoding one or more proteins capable of metabolizing a renewable carbon source to produce, and in some examples secrete, a fatty acid derivative. The modified hydrogen oxidising bacteria can then be used in a fermentation process to produce useful fatty acids and/or derivatives thereof using the renewable carbon source (biomass) as a starting material. In some examples, existing genetically tractable hydrogen oxidising bacteria are used because of the ease of engineering its pathways for controlling growth, production and reducing or eliminating side reactions that reduce biosynthetic pathway efficiencies. Also, such modified hydrogen oxidising bacteria can be used to consume renewable carbon sources in order to generate fuels that can be directly used as biofuels, without the need for special methods for storage, or transportation. In other examples, microorganisms that naturally produce hydrocarbons are engineered to overproduce hydrocarbons by expressing exogenous nucleic acid sequences that increase fatty acid production.

The method according to any aspect of the present invention may be used to produce fatty acid and/or derivatives thereof having defined carbon chain length, branching, and saturation levels. In particular examples, the production of homogeneous products decreases the overall cost associated with fermentation and separation. In particular, the fatty acid comprises at least 5 carbon atoms. For example, the fatty acid may be saturated or unsaturated. The fatty acid may comprise 5, 6, 7, 8, 9, 10, 11, 12, 13, 14, 15, 16, 17, 18, 19, 20 carbon atoms. In some examples, the fatty acid may comprise more than 20 carbon atoms. More in particular, the fatty acid and/or derivatives thereof may comprise a carbon chain that is at least 8, 10, 12, 14, 16, 18, 20, 22, 24, 26, 28, 30, 32, or 34 carbons long. In some examples at least 50%, 60%, 70%, 80%, 85%, 90%, or 95% of the fatty acid and/or derivative product made contains a carbon chain that is 8, 10, 12, 14, 16, 18, 20, 22, 24, 26, 28, 30, 32, or 34 carbons long. In yet other examples, at least 60%, 70%, 80%, 85%, 90%, or 95% of the fatty acid and/or derivative product contain 1, 2, 3, 4, or 5, points of unsaturation. In particular, the method according to any aspect of the present invention may result in the production of a combination of fatty acids. More in particular, the result may comprise a combination of at least 2, 3, 4, 5 or 6 fatty acids produced.

According to any aspect of the present invention, the carbon source used in step (b) may be any renewable carbon source. In particular, the carbon source may comprise H₂, CO₂ and/or O₂. More in particular, the carbon source comprises oxygen gas allowing for production of fatty acids in aerobic conditions.

The partial pressure of hydrogen in the H₂, CO₂ and O₂ gas introduced in step (b) may be 0.1 to 100 bar, 0.2 to 10 bar, particularly 0.5 to 4 bar. The partial pressure of carbon dioxide in the H₂, CO₂ and O₂ containing gas introduced in step (b) may be 0.03 to 100 bar, particularly 0.05 to 1 bar, more in particular 0.05 to 0.3 bar. The partial pressure of oxygen in the H₂, CO₂ and O₂ gas introduced in step (b) may be 0.001 to 100 bar, particularly 0.04 to 1 bar, more in particular 0.04 to 0.5 bar. In particular, the source of H₂ and/or CO₂ in step (b) may be synthesis gas. The synthesis gas may be used in combination with oxygen to be the source of H₂, CO₂ and O₂ gas introduced in step (b). More in particular, the H₂, CO₂ and O₂ gas introduced in step (b) according to any aspect of the present invention may comprise synthesis gas.

Synthesis gas can be provided from the by-product of coal gasification, for example. Consequently, the hydrogen oxidising bacterium converts a substance that is a waste product, into a valuable raw material.

Alternatively, synthesis gas may be provided by the gasification of widely available, low-cost agricultural raw materials for the process according to any aspect of the present invention. There are numerous examples of raw materials that can be converted into synthesis gas, as almost all forms of vegetation can be used for this purpose. Examples of raw materials are perennial grasses such as miscanthus, corn residues, processing waste such as sawdust. In general, the synthesis gas may be obtained in a gasification apparatus of dried biomass with primary products such as H₂, CO₂ and O₂, mainly through pyrolysis, partial oxidation and/or steam reforming. Usually a portion of the product gas is processed in order to optimize product yields, and to avoid formation of tar. Cracking of the undesired tar and CO in the synthesis gas may be carried out using lime and/or dolomite. These processes are described in detail in Reed, TB, 1981. Mixtures of different sources for the generation of synthesis gas can also be used.

In particular, the carbon contained in the synthesis gas in step (b) comprises at least 50% by weight, or at least 70%, 90% by weight of the carbon of all carbon sources that is available to the hydrogen oxidising bacteria in process step (b), wherein the weight percent of coal substance relate to the carbon atoms. Other carbon sources besides CO₂ from the synthesis gas may be available to the hydrogen oxidising bacteria, for example in the form of carbohydrates in the aqueous medium.

More CO₂ sources such as flue gas, petroleum refinery gases, product of yeast fermentation or clostridial fermentation, exhaust gases from the gasification of cellulose-containing materials or coal gasification and the like may be used as the source of CO₂ in the H₂, CO₂ and O₂ gas introduced in step (b).

In particular, the gas comprising H₂, CO₂ and/or O₂ introduced in step (b) comprises a weight ratio of 20 to 70: 10 to 45: 5 to 35 respectively. The ratio of H₂ and O₂ may vary depending on the source. For example, the weight ratio of H₂, to O₂ may be 2:1, 4:1, 5:1 and the like.

The term "hydrogen oxidising bacterium" may be used interchangeably with the term "knallgas bacterium", and refers to any bacterium capable of oxidising hydrogen, using oxygen as a terminal electron acceptor, and of fixing carbon dioxide under aerobic conditions. In particular, the knallgas bacterium may be selected from the group consisting of *Methanobacterium, Acetobacterium, Desulfovibrio, Desulfomonas, Paracoccus, Achromobacter, Alcaligenes, Pseudomonas, Nocardia* and *Cupriavidus.* More in particular, the knallgas bacterium may be a *Cupriavidus* strain, even more in particular, *Cupriavidus necator* H16. Exemplary knallgas bacteria may comprise of, but are not limited to *Acidovorax facilis, Acidovorax sp., Alcaligenes eutropha, Alcaligenes sp., Bradyrhizobium japonicum, Bradyrhizobium sp., Cupriavidus necator DSM 531, Heliobacter sp., Hydrogenobacter sp., Hydrogenobacter thermophilus, Hydrogenomonas eutropha, Hydrogenomonas pantotropha, Hydrogenomonas sp., Hydrogenomonas facilis, Hydrogenophaga sp., Hydrogenovibrio marinus (strain MH-110), Hydrogenovibrio sp., Oxyhydrogen microorganism, Pseudomonas hydrogenothermophila, Pseudomonas hydrogenovora, Pseudomonas sp., Ralstonia eutropha, Ralstonia sp., Rhizobium japonicum, Rhizobium sp., Rhodococcus opacus, Rhodococcus sp., Rhodopseudomonas acidophila, Rhodopseudomonas blastica, Rhodopseudomonas capsulata, Rhodopseudomonas palustris, Rhodopseudomonas sheroides, Rhodopseudomonas sulfoviridis, Rhodopseudomonas viridis, Rhodoseudomonas sp., Rhodospirillum rubrum, Rhodospirillum sp., Thiocapsa roseopersicina, Thiocapsus sp., Variovorax paradoxus, Variovorax sp., Xanthobacter sp., Hydrogenothermus marinus, Ralstonia eutropha, Cupriavidus necator, Alcaligenes eutrophus, Alcaligenes paradoxus, Paracoccus denitrificans, Pseudomonas facilis, Pseudomonas flava, Pseudomonas palleronii, Pseudomonas saccharophila, and Rhodococcus sp. (Nocardia opaca), Pseudomonas pseudoflava.* In one example, the knallgas bacterium may be a bacterium genetically modified to be a knallgas bacterium, whilst the corresponding wild type strain is not. In this case, the bacterium may be any organism amenable to such modifications, for example *E. coli.*

The phrase "increased activity of an enzyme", as used herein is to be understood as increased intracellular activity. Basically, an increase in enzymatic activity can be achieved by increasing the copy number of the gene sequence or gene sequences that code for the enzyme, using a strong promoter or employing a gene or allele that code for a corresponding enzyme with increased activity and optionally by combining these measures. Genetically modified cells or organisms used according to any aspect of the present invention are for example produced by transformation, transduction, conjugation or a combination of these methods with a vector that contains the desired gene, an allele of this gene or parts thereof and a vector that makes expression of the gene possible. Heterologous expression is in particular achieved by integration of the gene or of the alleles in the chromosome of the cell or an extra-chromosomally replicating vector.

The genetically modified cell may be genetically different from the wild type cell. The genetic difference between the genetically modified cell according to any aspect of the present invention and the wild type cell may be in the presence of a complete gene, amino acid, nucleotide etc. in the genetically modified cell that may be absent in the wild type cell. In one example, the genetically modified cell according to any aspect of the present invention may comprise enzymes that enable the cell to produce at least one fatty acid and/or acyl coenzyme A thereof; and convert the fatty acid and/or acyl coenzyme A thereof to the fatty acid ester. The wild type cell relative to the genetically modified cell of the present invention may have none or no detectable activity of the enzymes that enable the genetically modified cell to produce at least one fatty acid and/or derivative thereof.

The phrase "wild type" as used herein in conjunction with a cell may denote a cell with a genome make-up that is in a form as seen naturally in the wild. The term may be applicable for both the whole cell and for individual genes. The term "wild type" therefore does not include such cells or such genes where the gene sequences have been altered at least partially by man using recombinant methods.

A skilled person would be able to use any method known in the art to genetically modify a cell. According to any aspect of the present invention, the genetically modified cell may be genetically modified so that in a defined time interval, within 2 hours, in particular within 8 hours or 24 hours, it forms at least twice, especially at least 10 times, at least 100 times, at least 1000 times or at least 10000 times more fatty acid and/or thereof than the wild-type cell. The increase in product formation can be determined for example by cultivating the cell according to any aspect of the present invention and the wild-type cell each separately under the same conditions (same cell density, same nutrient medium, same culture conditions) for a specified time interval in a suitable nutrient medium and then determining the amount of target product (fatty acid, acyl coenzyme A thereof and the respective fatty acid ester) in the nutrient medium.

According to any aspect of the present invention, the hydrogen oxidising bacterium may be genetically modified relative to the wild type bacterium to increase the production of acyl-ACP or acyl-CoA, reduce the catabolism of fatty acid derivatives and intermediates, or to reduce feedback inhibition at specific points in the biosynthetic pathway. In one example, cellular resources can also be diverted to aid in the overproduction of fatty acids, for example the lactate, succinate and/or acetate pathways can be attenuated, and acetyl-CoA carboxylase (ACC) can be overexpressed. In particular, the expression of enzyme E₁ that may be capable of catalysing the conversion of acetyl CoA to acyl ACP via malonyl CoA may be increased in the hydrogen oxidising bacterium according to any aspect of the present invention. In one example, the hydrogen oxidising bacterium according to any aspect of the present invention may also comprise an increase in the expression of enzyme E₂ that may be capable of catalysing the conversion of Acyl ACP to the fatty acid. The the hydrogen oxidising bacterium according to any aspect of the present invention may comprise increase in the expression of E₁ and E₂.

The genetically modified hydrogen oxidising bacterium according to any aspect of the present invention may be cultured in a desired environment, for example one with limited glycerol (less than 1% w/v in the culture medium). As such, these hydrogen oxidising bacteria will have increased acetyl-CoA production levels. With an increase in acetyl-CoA production levels, malonyl-CoA production may also be increased by genetically modifying the hydrogen oxidising bacteria as described above, with DNA encoding accABCD (acetyl CoA carboxylase, for example accession number AAC73296, EC 6.4.1.2) included in the plasmid synthesized de novo. Fatty acid overproduction can be achieved by further including DNA encoding lipase (for example accession numbers CAA89087, CAA98876) in the plasmid synthesized de novo.

In some examples, acetyl-CoA carboxylase (ACC) is over-expressed to increase the intracellular concentration thereof by at least 2-fold, such as at least 5-fold, or at least 10-fold, for example relative to native expression levels.

In one example, the *plsB* (for example accession number AAC77011) D311 E mutation can be used to remove limitations on the pool of acyl-CoA.

Overexpression of a *sfa* gene (suppressor of Fab A, for example with accession number AAN79592) may also be included in the genetically modified hydrogen oxidising bacterium to increase production of monounsaturated fatty acids (Rock et al, 1996).

In particular, E₁ may be selected from a group of enzymes that are capable of catalysing the conversion of acetyl CoA to acyl ACP via malonyl CoA. E₁ may thus be selected from the group consisting of E₁ₐ accA (EC 6.4.1.2), E_{1b} accB (EC 6.4.1.2), E_{1c} accC (EC 6.3.4.14), E_{1d} accD (EC 6.4.1.2) and combinations thereof. More in particular, the genetically modified hydrogen oxidising bacterium according to any aspect of the present invention may be genetically modified to increase the expression of E₁ₐ, E_{1b}, E_{1c}, E_{1d}, E₁ₐE_{1b}, E₁ₐE_{1c}, E₁ₐE_{1d}, E_{1b}E_{1c}, E_{1b}E_{1d}, E_{1c}E_{1d}, E₁ₐE_{1b}E_{1c}, E₁ₐE_{1b}E_{1d}, E_{1b}E_{1c}E_{1d} or E₁ₐE_{1b}E_{1c}E_{1d}.

In particular, the genetically modified hydrogen oxidising bacterium according to any aspect of the present invention may be modified to overexpress at least one enzyme E₂ capable of catalysing the conversion of acyl ACP to fatty acid, possibly free fatty acid. More in particular, E₂ may be a thioesterase. Even more in particular, E₂ may be selected from the group consisting of acyl-ACP thioesterase E₂ₐ, acyl-CoA thioesterase E_{2b}, acyl-thioesterase E_{2c} and the like. In particular, E₂ₐ (EC 3.1.2.14 or EC 3.1.2.22) may be any enzyme that is capable of catalysing the hydrolysis of acyl-ACP thioester; E_{2b} (EC 3.1.2.2, EC 3.1.2.18, EC 3.1.2.19, EC 3.1.2.20 or EC 3.1.2.22) may be any enzyme that is capable of catalysing the hydrolysis of acyl-CoA thioester; E_{2c} (EC 3.1.2.2, EC 3.1.2.4, EC 3.1.2.18, EC 3.1.2.19, EC 3.1.2.20 or EC 3.1.2.22) may be any enzyme that is capable of catalysing the conversion of an acyl-thioester with an alcohol to a carboxylic acid ester. E₂ may be chosen to provide homogeneous products.

More in particular, E₂ may be E₂ₐ. For example, C₁₀ fatty acid and derivatives thereof can be produced by attenuating thioesterase C₁₈ (for example accession numbers AAC73596 and POADA1), which uses C18:1-ACP and expressing thioesterase C₁₀ (for example accession number Q39513), which uses C10-ACP. Thus, resulting in a relatively homogeneous population of fatty acids and/or derivatives thereof that have a carbon chain length of 10. In another example, C₁₄ fatty acid and derivatives can be produced by attenuating endogenous thioesterases that produce non-C₁₄ fatty acids and expressing the thioesterase with accession number Q39473, which uses C14-ACP). In yet another example, C₁₂ fatty acid and derivatives thereof can be produced by expressing thioesterases that use C12-ACP (for example accession number Q41635) and attenuating thioesterases that produce non-C12 fatty acids. Examples of E₂ that may be used to produce specific fatty acids are provided in Table 1 below. Acetyl CoA, malonyl CoA, and fatty acid overproduction can be verified using methods known in the art, for example by using radioactive precursors, HPLC, and GC-MS subsequent to cell lysis.

**Table 1. Examples of thioesterases for production of fatty acid from acyl-ACP**

| Accession Number | *Source Organism* | *Gene* | Preferential product produced |
|---|---|---|---|
| AAC73596 | *E*. *coli* | *tesA* without leader sequence | C18:1 |
| Q41635 | *Umbellularia california* | *fatB* | C12:0 |
| Q39513; | *Cuphea hookeriana* | *fatB2* | C8:0-C10:0 |
| AAC49269 | *Cuphea hookeriana* | *fatB3* | C14:0 - C16:0 |
| Q39473 | *Cinnamonum camphorum* | *fatB* | C14:0 |
| CAA85388 | *Arabidopsis thaliana* | *fatB*[M141T]* | C16:1 |
| NP 189147; NP 193041 | *Arabidopsis thaliana* | *fatA* | C18:1 |
| CAC39106 | *Bradyrhiizobium* | *fatA* | C18:1 |
| AAC72883 | *Cuphea hookeriana* | *fatA* | C18:1 |

According to any aspect of the present invention, the hydrogen oxidising bacterium may be genetically modified to express E₂ selected from the group consisting of E₂ₐ, E_{2b}, and E_{2c} in combination with E₁ₐ, E_{1b}, E_{1c}, E_{1d}, E₁ₐE_{1b}, E₁ₐE_{1c}, E₁ₐE_{1d}, E_{1b}E_{1c}, E_{1b}E_{1d}, E_{1c}E_{1d}, E₁ₐE_{1b}E_{1c}, E₁ₐE_{1b}E_{1d}, E_{1b}E_{1c}E_{1d} or E₁ₐE_{1b}E_{1c}E_{1d}. More in particular, the hydrogen oxidising bacterium may be genetically modified to express E₁ₐE_{1b}E_{1c}E_{1d}E₂ₐ.

In one example, E₁ₐ, has sequence identity of at least 50% to a polypeptide of AAC73296 or EG11647; E_{1b} has sequence identity of at least 50% to a polypeptide of EG10275; E_{1c} has sequence identity of at least 50% to a polypeptide of EG10276; and/or E_{1d} has sequence identity of at least 50% to a polypeptide of EG10217 and combinations thereof. E₁ₐ E_{1b}. E_{1c} and E_{1d} used according to any aspect of the present invention may comprise sequence identity of at least 60, 65, 70, 75, 80, 85, 90, 95, 98 or 100% to a polypeptide of any one of sequences of accA (AAC73296 or EG11647), accB (EG10275), accC (EG10276), accD (EG10217) and combinations thereof. In particular, the hydrogen oxidising bacterium may be genetically modified to at least comprise a functional fragment of any of the polypeptides for catalysing the conversion of conversion of acetyl CoA to acyl ACP via malonyl coA.

In one example, E₂ has sequence identity of at least 50% to a polypeptide selected from the group consisting of AAC79596, ACC49269, CAA58388, NP189147, NP193041, , AAC72883, AAC72881.1, ABB71579.1, CAC19934.1, AAC49180.1 (encoded by SEQ ID No.10), AAC49783.1, AAC49179.1, CAB60830.1, ABB71581.1, AAC49269.1, CAC19933.1, CAA54060.1, AAC72882.1, Q39513.1, AAC49784.1, ABO38558.1, ABO38555.1, ABO38556.1, ABO38554.1, ADB79568.1, ADB79569.1, ACQ57188.1, ACQ57189.1, ABK96561.1, ACQ63293.1, ACQ57190.1, Q9SQI3.1, ABU96744.1, ABC47311.1, XP_002324962.1, AAD01982.1, AAB51525.1, ACV40757.1, XP_002309244.1, CBI28125.3, ABD91726.1, XP_002284850.1, XP_002309243.1, XP_002515564.1, ACR56792.1, ACR56793.1, XP_002892461.1, ABI18986.1, NP_172327.1, CAA85387.1, CAA85388.1, ADA79524.1, ACR56795.1, ACR56794.1, CAN81819.1, ACF17654.1, AAB71729.1, ABH11710.1, ACQ57187.1, AAX51637.1, AAB88824.1, AAQ08202.1, AAB71731.1, AAX51636.1, CAC80370.1, CAC80371.1, AAG43858.1, ABD83939.1, AAD42220.2, AAG43860.1, AAG43861.1, AAG43857.1, AAL15645.1, AAB71730.1, NP_001068400.1, EAY86877.1, NP_001056776.1, XP_002436457.1, NP_001149963.1, ACN27901.1, EAY99617.1, ABL85052.1, XP_002437226.1, NP_001151366.1, ACF88154.1, NP_001147887.1, XP_002453522.1, BAJ99650.1, EAZ37535.1, EAZ01545.1, AAN17328.1, EAY86884.1, EEE57469.1, Q41635.1, AAM09524.1, Q39473.1, NP_001057985.1, AAC49001.1, XP_001752161.1, XP_001770108.1, XP_001784994.1, XP_002318751.1, NP_001047567.1, XP_002322277.1, XP_002299627.1, XP_002511148.1, CB115695.3, XP_002299629.1, XP_002280321.1, CAN60643.1, XP_002459731.1, XP_002975500.1, XP_002962077.1, XP_001773771.1, NP_001151014.1, XP_002317894.1, XP_002971008.1, XP_001774723.1, XP_002280147.1, XP_002526311.1, XP_002517525.1, XP_001764527.1, ABI20759.1, BAD73184.1, XP_002987091.1, XP_002985480.1, CBI26947.3, ABI20760.1, XP_002303055.1, XP_002885681.1, ADH03021.1, XP_002532744.1, EAY74210.1, EEC84846.1, EEE54649.1, AAG35064.1, AAC49002.1, CAD32683.1, ACF78226.1, BAJ96402.1, XP_002462626.1, NP_001130099.1, XP_002462625.1, ABX82799.3, Q42712.1, NP_193041.1, AAB51524.1, NP_189147.1, ABR18461.1, XP_002863277.1, AAC72883.1, AAA33019.1, CBI40881.3, XP_002262721.1, AAB51523.1, NP_001063601.1, ADB79567.1, AAL77443.1, AAL77445.1, AAQ08223.1, AAL79361.1, CAA52070.1, AAA33020.1, CAA52069.1, XP_001785304.1, CAC39106.1, XP_002992591.1, XP_002968049.1, XP_001770737.1, XP_001752563.1, AAG43859.1, XP_002978911.1, XP_002977790.1, ACB29661.1, XP_002314829.1, XP_002991471.1, EAZ45287.1, XP_002986974.1, EEC73687.1, XP_002312421.1, ACJ84621.1, NP_001150707.1, AAD28187.1, XP_001759159.1, XP_001757193.1, XP_002322077.1, ABE01139.1, XP_002447294.1, AAX54515.1, AAD33870.1. In particular, E₂ may be selected from the group consisting of AAC72881.1, ABB71579.1, CAC19934.1, AAC49180.1, AAC49783.1, AAC49179.1, CAB60830.1, ABB71581.1, AAC49269.1, CAC19933.1, CAA54060.1, AAC72882.1, Q39513.1, AAC49784.1, AAC72883.1, Q41635.1, and AAC49001.1 and combinations thereof. E₂ used according to any aspect of the present invention may comprise sequence identity of at least 60, 65, 70, 75, 80, 85, 90, 95, 98 or 100% to a polypeptide of any one of sequences of provided above and combinations thereof. In particular, the hydrogen oxidising bacterium may be genetically modified to at least comprise a functional fragment of any of the polypeptides for catalysing the conversion of conversion of acyl ACP to at least one fatty acid.

In some examples, the fatty acid may undergo a further to step to produce at least one fatty acid derivative selected from the group consisting of butanol, fatty alcohols, fatty acid esters, hydrocarbons, wax esters and the like.

In one example, the genetically modified hydrogen oxidising bacterium according to any aspect of the present invention may be modified to overexpress at least one wax ester synthase (EC 2.3.1.75) (E₂₁). The genetically modified hydrogen oxidising bacterium may be modified to express E₁ₐE_{1b}E_{1c}E_{1d}E₂E₂₁. In another example, the genetically modified hydrogen oxidising bacterium according to any aspect of the present invention may be modified to overexpress at least one alcohol acetyltransferase (2.3.1.84) (E₂₂). In yet other examples, the genetically modified hydrogen oxidising bacterium may be modified to express E₁ₐE_{1b}E_{1c}E_{1d}E₂E₂₁E₂₂, E₁ₐE_{1b}E_{1c}E_{1d}E₂E₂₂ and the like.

In yet other examples, the genetically modified hydrogen oxidising bacterium according to any aspect of the present invention may be modified to overexpress at least one acyl-CoA reductase (EC 1.2.1.50) (E₂₃) and at least one alcohol dehydrogenase (EC 1.1.1.1) (E₂₄). In particular, the genetically modified hydrogen oxidising bacterium may be modified to express E₁ₐE_{1b}E_{1c}E_{1d}E₂E₂₃E₂₄, E₁ₐE_{1b}E_{1c}E_{1d}E₂E₂₃, E₁ₐE_{1b}E_{1c}E_{1d}E₂E₂₄ and the like.

In a further example, the genetically modified hydrogen oxidising bacterium according to any aspect of the present invention may be modified to overexpress at least one fatty alcohol forming acyl-CoA reductase (1.1.1.*) (E₂₅). In particular, the genetically modified hydrogen oxidising bacterium may be modified to express E₁ₐE_{1b}E_{1c}E_{1d}E₂E₂₅, and the like.

In one example, the genetically hydrogen oxidising bacterium according to any aspect of the present invention may be modified to increase the expression relative to the wild type bacterium of enzyme E₂₆ a fatty acid methyl transferase (EC 2.1.1.15). In particular, the genetically modified hydrogen oxidising bacterium may be modified to express E₁ₐE_{1b}E_{1c}E_{1d}E₂E₂₆ and the like.

The genetically modified hydrogen oxidising bacterium according to any aspect of the present invention may also be modified to have one or more endogenous genes functionally deleted or attenuated to aid in the production of fatty acids and/or derivatives thereof. For example, *ackA* (EC 2.7.2.1), *ackB* (EC2.7.2.1), *adhE* (EC 1.1.1.1,1.2.1.10), *fabF* (EC2.3.1.179), *fabR* (accession NP_418398), *fadE* (EC 1.3.99.3, 1.3.99.-), GST (EC 6.3.2.3), *gpsA* (EC 1.1.1.94), *IdhA* (EC 1.1.1.28), *pflB* (EC EC 2.3.1.54), *plsB* (EC 2.3.1.15), *poxb* (EC 1.2.2.2), *pta* (EC 2.3.1.8), glutathione synthase (EC 6.3.2.3) and combinations thereof.

The genetically modified hydrogen oxidising bacterium according to any aspect of the present invention may also be modified to have one or more endogenous genes overexpressed. For example, *pdh, panK, aceEF* (encoding the Elp dehydrogenase component and the E2p dihydrolipoamide acyltransferase component of the pyruvate and 2-oxoglutarate dehydrogenase complexes, Accession Numbers: NP_414656, NP_414657, EC: 1.2.4.1. 2.3.1.61, 2.3.1.12), *accABCD*/*fabH*/*fabD*/*fabG*/*acpP*/*fabF* (Accession Numbers: Q0KCA7, G0EWI2, F8GYI0, G0EWI3, F8GWN5, GOERC9, CAD85557, CAD85558, NP_842277, NP_841683, NP_415613, EC 6.4.1.2 C, EC: 2.3.1.180, 2.3.1.39, 1.1.1.100, 1.6.5.3, 2.3.1.179), genes encoding fatty-acyl-coA reductases (Accession numbers: AAC45217, EC 1.2.1.-), *UdhA* or similar genes (encoding pyridine nucleotide transhydrogenase, Accession numbers: CAA46822 , EC: 1.6.1.1) and genes encoding fatty-acyl-coA reductases (Accession numbers: AAC45217, EC 1.2.1.-).

Fatty acid synthase (FAS) is a group of peptides that catalyse the initiation and elongation of acyl chains (Marrakchi et al., 2002). The acyl carrier protein (ACP) along with the enzymes in the FAS pathway control the length, degree of saturation and branching of the fatty acids produced. Enzymes that can be included in FAS include but are not limited to AccABCD, FabD, FabH, FabG, FabA, FabZ, Fabl, FabK, FabL, FabM, FabB, FabF and the like. The genes may be overexpressed or attenuated depending on the specific fatty acid desired to provide the suitable conditions for production of the fatty acid. The precursors of fatty acid production according to any aspect of the present invention are acetyl-CoA and malonyl-CoA. Hydrogen oxidising bacterium engineered to overproduce these components can serve as the starting point for subsequent genetic engineering steps to provide fatty acid derivatives such as, fatty acid esters, hydrocarbons, fatty alcohols and the like. Several different modifications can be made, either in combination or individually, to the host hydrogen oxidising bacterium strain to obtain increased acetyl CoA/malonyl CoA/fatty acid and fatty acid derivative production. For example, to increase acetyl CoA production, a plasmid with *pdh, panK, aceEF,* (encoding the Elp dehydrogenase component and the E2p dihydrolipoamide acyltransferase component of the pyruvate and 2-oxoglutarate dehydrogenase complexes), *fabH*/*fabD*/*fabG*/*acpP*/*fabF,* and in some examples additional DNA encoding fatty-acyl-CoA reductases and aldehyde decarbonylases, all under the control of a constitutive, or otherwise controllable promoter, can be constructed. Exemplary Genbank accession numbers for these genes are: *pdh* (BAB34380, AAC73227, AAC73226), *panK* (also known as coaA, AAC76952), (E₃ and E₄) *aceEF* (AAC73227, AAC73226), (E₁₂) *fabH* (AAC74175), (E₁₀) *fabD* (AAC74176), (E₁₁) *fabG* (AAC74177), (E₅) *acpP* (AAC74178) and *fabF* (AAC74179).

Other enzymes such as *fadE, gpsA, IdhA, pflb, adhE, pta, poxB, ackA,* and/or *ackB* may be knocked-out, or their expression levels can be reduced, in the engineered hydrogen oxidising bacterium according to any aspect of the present invention by transformation with conditionally replicative or non-replicative plasmids containing null or deletion mutations of the corresponding genes, or by substituting promoter or enhancer sequences. Exemplary Genbank accession numbers for these genes are: *fadE* (AAC73325), *gspA* (AAC76632), *IdhA* (AAC74462), *pflb* (AAC73989), *adhE* (AAC74323), *pta* (AAC75357), *poxB* (AAC73958), *ackA* (AAC75356), and *ackB* (BAB81430).

Even more in particular, the genetically modified hydrogen oxidising bacterium according to any aspect of the present invention may be modified to overexpress at least one enzyme selected from the group consisting of E₃ *aceE* (EC 1.2.4.1, 2.3.1.61, 2.3.1.12), E₄ aceF (EC 1.2.4.1, 2.3.4.16, 2.3.1.12), E₅ *acpP* (AAC74178), E₆ *fadD* (EC 2.3.1.86), E₇ *cerl* (EC 4.1.99.5), E₈ *fabA* (EC4.2.1.60), E₉ *fabB* (EC 2.3.1.41), E₁₀ *fabD* (EC 2.3.1.39), E₁₁ *fabG* (EC 1.1.1.100), E₁₂ *fabH* (EC 2.3.1.180), E₁₃ *fabI* (EC 1.3.1.9), E₁₄ *fabZ* (EC 4.2.1.-), E₁₅ lipase (EC 3.1.1.3), E₁₆ malonyl-CoA decarboxylase (EC 4.1.1.9, 4.1.1.41), E₁₇ *panD* (EC 4.1.1.11), E₁₈ *panK* (EC 2.7.1.33), E₁₉ *pdh* (EC 1.2.4.1), E₂₀ *udhA* (EC 1.6.1.1) and combinations thereof. In one example, the genetically modified hydrogen oxidising bacterium according to any aspect of the present invention may be modified to overexpress E₁ₐ, E_{1b}, E_{1c} and/or E_{1d} and combinations thereof as mentioned above in combination with at least one enzyme selected from E₃ E₄ E₅ E₆, E₇, E₈, E₉, E₁₀, E₁₁, E₁₂, E₁₃, E₁₄, E₁₅, E₁₆, E₁₇, E₁₈, E₁₉ and E₂₀. In one example, the hydrogen oxidising bacterium may be genetically modified to express E₁ₐE_{1b}E_{1c}E_{1d} and at least one enzyme selected from E₃ to E₂₀ or all enzymes E₃ to E₂₀. In particular, the hydrogen oxidising bacterium according to any aspect of the present invention may overexpress E₁ₐE_{1b}E_{1c}E_{1d} in combination with at least E₆.

Unless otherwise mentioned, all technical and scientific terms used herein have the same meaning as commonly understood by a skilled person. Any method known in the art may be used by the skilled person. Some examples of these methods are provided in the present description. The materials, methods, and examples are illustrative only and not intended to be limiting.

The accession numbers used throughout this description are derived from the NCBI database (National Centre for Biotechnology Information) maintained by the National Institute of Health, U. S. A. The accession numbers are as provided in the database on 30 June 2014. Similarly, the EC numbers provided throughout this description are derived from the KEGG Ligand database, maintained by the Kyoto Encyclopedia of Genes and Genomics, sponsored in part by the University of Tokyo. The EC numbers are as provided in the database on 30 June 2014.

### EXAMPLES

The foregoing describes preferred embodiments, which, as will be understood by those skilled in the art, may be subject to variations or modifications in design, construction or operation without departing from the scope of the claims. These variations, for instance, are intended to be covered by the scope of the claims.

### Example 1

### Construction of plasmids for the preparation of fatty acids with Ralstonia eutropha

For the construction of plasmids for the production of fatty acids from R. *eutropha,* three synthetic expression cassettes were synthesized consisting of the following components:
1. The ribosome binding site of the R. *eutropha groEL* gene (SEQ ID NO: 1), the gene encoding the thioesterase from *Cuphea hookeriana ChFATB2* (SEQ ID NO: 2) which has been 5'-truncated at the plastid targeting sequence, the terminator of the *E. coli rrnB* gene (SEQ ID NO: 5), wherein the coding region for *ChFATB2* in the translation of *R. eutropha* is codon optimised (RBS RegroEL-ChFATB2-T; SEQ ID NO: 6).
2. The ribosome binding site of the *R. eutropha groEL* gene (SEQ ID NO: 1), the gene encoding the thioesterase of *Cocos nucifera* CnFATB3 (SEQ ID NO:3), the terminator of the *E. coli rrnB* gene (SEQ ID NO: 5), wherein the coding region for *ChFATB3* in the translation of R. *eutropha* is codon optimised (RBS RegroEL-ChFATB3-T; SEQ ID NO: 7).
3. The ribosome binding site of the *R. eutropha groEL* gene (SEQ ID NO: 1), the gene encoding the thioesterase of *Umbellularia californica UcFATB1* (SEQ ID NO: 4) the terminator of the *E. coli rrnB gene* (SEQ ID NO: 5), wherein the coding region for *UcFATB1* in the translation of *R. eutropha* is codon optimised (RBS RegroEL-UCTE-T; SEQ ID NO: 8).

The expression cassettes RBS RegroEL-ChFATB2-T, RBS RegroEL-CnFATB3-T and RBS RegroEL-UcFATB1-T were then cloned via Kpnl / Hindlll in the broad host range expression vector pBBR1 MCS-2 (SEQ ID NO: 9), so that expression of the genes was under the control of the *E. coli* lacZ promoter. The resulting expression plasmids were designated as PbBr-ChFATB2, PbBr-CnFATB3 and PbBr-UcFATB1 with respective sequences, SEQ ID NOs: 10, 11 and 12.

### Example 2

### Introducing plasmids for the production of fatty acids in Ralstonia eutropha

The plasmids from above were transfected into competent *E. coli* S17-1 cells, a strain where the conjugative transfer of plasmids from *Ralstonia eutropha* among other strains is possible. For this purpose, a Spotmating conjugation (as in FRIEDRICH et al, 1981) with the respective plasmids was carried out where *E. coli* S17 -1 strain is the donor and R. eutropha H16 (reclassified as Alcaligenes eutrophus, DSMZ 428) and *R. eutropha* PHB-4 (reclassified as Alcaligenes eutrophus, DSMZ 541) the recipient. Transconjugants were obtained in all cases which carry the respective plasmids and the corresponding strains designated as follows:
- *R. eutropha* H16 PbBr-ChFATB2, *R. eutropha* H16 PbBr-CnFATB3, *R. eutropha* H16 PbBr-UcFATB1, R. *eutropha* PHB-4-PbBr ChFATB2, R. *eutropha* PHB-4-PbBr CnFATB3, and R. *eutropha* PHB-4-PbBr UcFATB1.

### Example 3

### Quantification of fatty acids

Quantification of octanoic acid, 3-hydroxydecanoic acid, decanoic acid, lauric acid, 3-hydroxymyristic acid, myristic acid, palmitoleic acid, palmitic acid, oleic acid and stearic acid in the fermentation samples is performed by HPLC-ESI / MS based on an internal calibration for all analytes and using the internal standard D3 lauric acid (methyl-D3, 99%) of octanoic acid, 3-hydroxydecanoic acid, decanoic acid, lauric acid, 3 hydroxymyristic acid, myristic acid, palmitoleic acid, stearic acid, and D3 (D3-methyl, 98%) of palmitic acid, oleic acid, stearic acid.

The following devices are used:
- HPLC system: Surveyor (Thermo Fisher Scientific, Waltham, Massachusetts, USA), consisting of Surveyor MS Pump, Surveyor Autosampler Plus and Surveyor Surveyor PDA
- mass spectrometer: TSQ Vantage HESI II - source (Thermo Fisher Scientific, Waltham, Massachusetts, USA)
- HPLC column: XBridge BEH C8, 100 x 2.1 mm, particle size: 2.5 microns, pore size 130 Å (Waters, Milford Massachusetts, USA)

The samples are prepared by mixing 1200 µl of acetone and 300 µl of the sample for about 10 seconds and then centrifuged at about 13,000 rpm for 5 min. The clear supernatant is removed and analysed after appropriate dilution with acetone. For each 900µl of the diluted sample 100 µl of ISTD solution is pipetted in.

HPLC separation is carried out using the above HPLC column. The injection volume is 2µl, the column temperature 25 °C, flow rate 0.3 mL/min. The mobile phase consists of eluent A (water + 10 mM ammonium acetate adjusted to pH = 9 neutralized with ammonia) and eluent B (acetonitrile / mobile phase A 95/5). The following gradient is used:

| **Time [min]** | **Eluent A [%]** | **Eluent B [%]** |
|---|---|---|
| 0 | 95 | 5 |
| 1 | 95 | 5 |
| 1,1 | 70 | 30 |
| 7 | 5 | 95 |
| 8 | 5 | 95 |

ESI-MS analysis is carried out in negative ionization with the following parameters from the ESI source:
- Spray Voltage: 3000 V
- Vaporizer Temperature: 380 ° C
- Sheath gas pressure: 40
- Aux Gas Pressure: 15
- Capillary Temperature: 380 ° C

The detection and quantification of individual compounds is made by 'single ion monitoring (SIM)' with the following parameters:

| **Analyte** | **Ion [M-H]⁻ [m/z]** | **Scan width [m/z]** | **Scan Time [ms]** | **Peak-Weite Q₃** |
|---|---|---|---|---|
| Octanic acid | 143,13 | 0,002 | 100 | 0,7 |
| 3-hydroxydecanoic acid | 187,13 | 0,002 | 50 | 0,7 |
| decanoic acid | 171,13 | 0,002 | 100 | 0,7 |
| lauric acid | 199,16 | 0,002 | 50 | 0,7 |
| 3-hydroxymyristic acid | 243,18 | 0,002 | 50 | 0,7 |
| myristic acid | 227,19 | 0,002 | 50 | 0,7 |
| Palmitoleinsaure | 253,18 | 0,002 | 50 | 0,7 |
| palmitoleic acid | 255,22 | 0,002 | 30 | 0,7 |
| oleic acid | 281,23 | 0,002 | 30 | 0,7 |
| stearic acid | 283,25 | 0,002 | 30 | 0,7 |
| D3- lauric acid | 202,16 | 0,002 | 50 | 0,7 |
| D3- stearic acid | 286,25 | 0,002 | 30 | 0,7 |

After cultivation of the following strains,the formation of fatty acids can be detected:
- *R. eutropha* H16 pBBR-ChFATB2
- *R. eutropha* H16 pBBR-CnFATB3
- *R. eutropha* H16 pBBR-UcFATB1
- *R. eutropha* PHB-4 pBBR-ChFATB2
- *R. eutropha* PHB-4 pBBR-CnFATB3
- *R. eutropha* PHB-4 pBBR-UcFATB1

### REFERENCES

Reed, TB, 1981, Biomass: principles and technology, Noves Data Corporation, Park Ridge, NJ. Marrakchi et al., Biochemical Society, 30:1050-1055, 2002
Rock et al, J. Bacteriology 178:5382-5387, 1996
FRIEDRICH et al, 1981, Naturally Occurring genetic transfer of hydrogen-oxidizing ability in between strains of Alcaligenes eutrophus J Bacteriol 147: 198-205

## Claims

1. A method of producing at least one fatty acid and/or derivative thereof from a gas comprising H₂, CO₂ and O₂ the method comprising the steps of:
(a) providing a genetically modified hydrogen oxidising bacterium in an aqueous medium; and
(b) contacting the aqueous medium with a gas comprising H₂, CO₂ and O₂ in a weight ratio of 20 to 70: 10 to 45: 5 to 35;
wherein the fatty acid comprises at least 5 carbon atoms and
wherein the hydrogen oxidising bacterium is genetically modified relative to the wild type bacterium to increase the expression of enzyme E₁ that is capable of catalysing the conversion of acetyl CoA to acyl ACP via malonyl CoA and to increase the expression of enzyme E₂ that is capable of catalysing the conversion of Acyl ACP to the fatty acid.

2. The method according to claim 1, wherein E₁ is selected from the group consisting of E₁ₐ accA (EC 6.4.1.2), E_{1b} accB (EC 6.4.1.2), E_{1c} accC (EC 6.4.1.2) and E_{1d} accD (EC 6.4.1.2) and combinations thereof, and/or E₂ is at least one thioesterase (EC 3.1.2.14 and EC 3.1.2.22).

3. The method according to either claim 1 or 2, wherein the hydrogen oxidising bacterium is genetically modified relative to the wild type bacterium to increase the expression of enzyme E₁ₐ, E_{1b}, E_{1c} and E_{1d}.

4. The method according to either claim 2 or 3, wherein
(a) E₁ₐ has sequence identity of at least 50% to a polypeptide with accession number AAC73296 or EG11647, and combinations thereof,
(b) E_{1b} has sequence identity of at least 50% to a polypeptide with accession number EG10275 and combinations thereof,
(c) E_{1c} has sequence identity of at least 50% to a polypeptide with accession number EG10276 and combinations thereof, and
(d) E_{1d} has sequence identity of at least 50% to a polypeptide of with accession number EG10217 and combinations thereof,
or to a functional fragment of any of the polypeptides for catalysing the conversion of conversion of acetyl CoA to acyl ACP via malonyl CoA.

5. The method according to any one of claims 2 to 4 wherein E₂ is an acyl-ACP thioesterase.

6. The method according to claim 5, wherein E₂ has sequence identity of at least 50% to a polypeptide with accession number selected from the group consisting of AAC72881.1, ABB71579.1, CAC19934.1, AAC49180.1, AAC49783.1, AAC49179.1, CAB60830.1, ABB71581.1, AAC49269.1, CAC19933.1, CAA54060.1, AAC72882.1, Q39513.1, AAC49784.1, AAC72883.1, Q41635.1, and AAC49001.1 and combinations thereof.

7. The method according to any of the preceding claims, wherein the hydrogen oxidising bacterium is further genetically modified relative to the wild type bacterium to increase the expression of at least one enzyme selected from the group consisting of E₃ *aceE* (EC 1.2.4.1, 2.3.1.61, 2.3.1.12), E₄ aceF (EC 1.2.4.1, 2.3.4.16, 2.3.1.12), E₅ *acpP* (AAC74178), E₆ *fadD* (EC 2.3.1.86), E₇ *cerI* (EC 4.1.99.5), E₈ *fabA* (EC4.2.1.60), E₉ *fabB* (EC 2.3.1.41), E₁₀ *fabD* (EC 2.3.1.39), E₁₁ *fabG* (EC 1.1.1.100), E₁₂ *fabH* (EC 2.3.1.180), E₁₃ *fabI* (EC 1.3.1.9), E₁₄ fabZ(EC 4.2.1.-), E₁₅ lipase (EC 3.1.1.3), E₁₆ malonyl-CoA decarboxylase (EC 4.1.1.9,4.1.1.41), E17 *panD* (EC 4.1.1.11), E₁₈ *panK* (EC 2.7.1.33), E₁₉ *pdh* (EC 1.2.4.1), E₂₀ *udhA* (EC 1.6.1.1) and combinations thereof.

8. The method according to any of the preceding claims, wherein the hydrogen oxidising bacteria is selected from the group consisting of *Achromobacter, Acidithiobacillus, Acidovorax, Alcaligenes, Anabena, Aquifex, Arthrobacter, Azospirillum, Bacillus, Bradyrhizobium, Cupriavidus, Derxia, Helicobacter, Herbaspirillum, Hydrogenobacter, Hydrogenobaculum, Hydrogenophaga" Hydrogenophilus, Hydrogenothermus, Hydrogenovibrio, Ideonella sp.* 01, *Kyrpidia, Metallosphaera, Methanobrevibacter, Myobacterium, Nocardia, Oligotropha, Paracoccus, Pelomonas, Polaromonas, Pseudomonas, Pseudonocardia, Rhizobium, Rhodococcus, Rhodopseudomonas, Rhodospirillum, Streptomyces, Thiocapsa, Treponema,Variovorax, Xanthobacter* and *Wautersia.*

9. The method according to any of the preceding claims, wherein the hydrogen oxidising bacterium is further genetically modified to increase the expression relative to the wild type bacterium of enzyme E₂₁ a wax ester synthase (EC 2.3.1.75).

10. The method according to any of the preceding claims, wherein the hydrogen oxidising bacterium is further genetically modified to increase the expression relative to the wild type bacterium of enzyme E₂₆ a fatty acid methyl transferase (EC 2.1.1.15).

11. The method according to any of the preceding claims, wherein the bacterium comprises an exogenous nucleic sequence encoding ACP, Sfa, or combinations thereof.


SEQUENCE LISTING

<110>  Evonik Industries AG

<120>  FATTY ACID AND DERIVATIVES PRODUCTION

<130>  2013P00096EP

<160>  12

<170>  PatentIn version 3.5

<210>  1
<211>  17
<212>  DNA
<213>  Ralstonia eutropha

<400>  1
tttcaggaga ttcaaga                                                    17


<210>  2
<211>  305
<212>  PRT
<213>  Cuphea hookeriana

<400>  2

Met His Asp Arg Lys Ser Lys Arg Pro Asp Met Leu Val Asp Ser Phe
1               5                   10                  15


Gly Leu Glu Ser Thr Val Gln Asp Gly Leu Val Phe Arg Gln Ser Phe
            20                  25                  30


Ser Ile Arg Ser Tyr Glu Ile Gly Thr Asp Arg Thr Ala Ser Ile Glu
        35                  40                  45


Thr Leu Met Asn His Leu Gln Glu Thr Ser Leu Asn His Cys Lys Ser
    50                  55                  60


Thr Gly Ile Leu Leu Asp Gly Phe Gly Arg Thr Leu Glu Met Cys Lys
65                  70                  75                  80


Arg Asp Leu Ile Trp Val Val Ile Lys Met Gln Ile Lys Val Asn Arg
                85                  90                  95


Tyr Pro Ala Trp Gly Asp Thr Val Glu Ile Asn Thr Arg Phe Ser Arg
            100                 105                 110


Leu Gly Lys Ile Gly Met Gly Arg Asp Trp Leu Ile Ser Asp Cys Asn
        115                 120                 125


Thr Gly Glu Ile Leu Val Arg Ala Thr Ser Ala Tyr Ala Met Met Asn
    130                 135                 140

Gln Lys Thr Arg Arg Leu Ser Lys Leu Pro Tyr Glu Val His Gln Glu
145                 150                 155                 160

Ile Val Pro Leu Phe Val Asp Ser Pro Val Ile Glu Asp Ser Asp Leu
                165                 170                 175

Lys Val His Lys Phe Lys Val Lys Thr Gly Asp Ser Ile Gln Lys Gly
            180                 185                 190

Leu Thr Pro Gly Trp Asn Asp Leu Asp Val Asn Gln His Val Ser Asn
        195                 200                 205

Val Lys Tyr Ile Gly Trp Ile Leu Glu Ser Met Pro Thr Glu Val Leu
    210                 215                 220

Glu Thr Gln Glu Leu Cys Ser Leu Ala Leu Glu Tyr Arg Arg Glu Cys
225                 230                 235                 240

Gly Arg Asp Ser Val Leu Glu Ser Val Thr Ala Met Asp Pro Ser Lys
                245                 250                 255

Val Gly Val Arg Ser Gln Tyr Gln His Leu Leu Arg Leu Glu Asp Gly
            260                 265                 270

Thr Ala Ile Val Asn Gly Ala Thr Glu Trp Arg Pro Lys Asn Ala Gly
        275                 280                 285

Ala Asn Gly Ala Ile Ser Thr Gly Lys Thr Ser Asn Gly Asn Ser Val
    290                 295                 300

Ser
305

<210>  3
<211>  327
<212>  PRT
<213>  Cocos nucifera

<400>  3

Met Pro Asp Trp Ser Met Leu Leu Ala Ala Ile Arg Thr Ile Phe Ser
1               5                   10                  15

Ala Ala Glu Lys Gln Trp Thr Leu Leu Asp Ser Lys Lys Arg Gly Ala
            20                  25                  30

Asp Ala Val Ala Asp Ala Ser Gly Val Gly Lys Met Val Lys Asn Gly
        35                  40                  45

Leu Val Tyr Arg Gln Asn Phe Ser Ile Arg Ser Tyr Glu Ile Gly Val
    50                  55                  60

Asp Lys Arg Ala Ser Val Glu Ala Leu Met Asn His Phe Gln Glu Thr
65                  70                  75                  80

Ser Leu Asn His Cys Lys Cys Ile Gly Leu Met His Gly Gly Phe Gly
                85                  90                  95

Cys Thr Pro Glu Met Thr Arg Arg Asn Leu Ile Trp Val Val Ala Lys
            100                 105                 110

Met Leu Val His Val Glu Arg Tyr Pro Trp Trp Gly Asp Val Val Gln
        115                 120                 125

Ile Asn Thr Trp Ile Ser Ser Ser Gly Lys Asn Gly Met Gly Arg Asp
    130                 135                 140

Trp His Val His Asp Cys Gln Thr Gly Leu Pro Ile Met Arg Gly Thr
145                 150                 155                 160

Ser Val Trp Val Met Met Asp Lys His Thr Arg Arg Leu Ser Lys Leu
                165                 170                 175

Pro Glu Glu Val Arg Ala Glu Ile Thr Pro Phe Phe Ser Glu Arg Asp
            180                 185                 190

Ala Val Leu Asp Asp Asn Gly Arg Lys Leu Pro Lys Phe Asp Asp Asp
        195                 200                 205

Ser Ala Ala His Val Arg Arg Gly Leu Thr Pro Arg Trp His Asp Phe
    210                 215                 220

Asp Val Asn Gln His Val Asn Asn Val Lys Tyr Val Gly Trp Ile Leu
225                 230                 235                 240

Glu Ser Val Pro Val Trp Met Leu Asp Gly Tyr Glu Val Ala Thr Met
                245                 250                 255

Ser Leu Glu Tyr Arg Arg Glu Cys Arg Met Asp Ser Val Val Gln Ser
            260                 265                 270

Leu Thr Ala Val Ser Ser Asp His Ala Asp Gly Ser Pro Ile Val Cys
        275                 280                 285

Gln His Leu Leu Arg Leu Glu Asp Gly Thr Glu Ile Val Arg Gly Gln
    290                 295                 300

Thr Glu Trp Arg Pro Lys Gln Gln Ala Arg Asp Leu Gly Asn Met Gly
305                 310                 315                 320

Leu His Pro Thr Glu Ser Lys
                325

<210>  4
<211>  301
<212>  PRT
<213>  Umbellularia californica

<400>  4

Met Thr Leu Glu Trp Lys Pro Lys Pro Lys Leu Pro Gln Leu Leu Asp
1               5                   10                  15

Asp His Phe Gly Leu His Gly Leu Val Phe Arg Arg Thr Phe Ala Ile
            20                  25                  30

Arg Ser Tyr Glu Val Gly Pro Asp Arg Ser Thr Ser Ile Leu Ala Val
        35                  40                  45

Met Asn His Met Gln Glu Ala Thr Leu Asn His Ala Lys Ser Val Gly
    50                  55                  60

Ile Leu Gly Asp Gly Phe Gly Thr Thr Leu Glu Met Ser Lys Arg Asp
65                  70                  75                  80

Leu Met Trp Val Val Arg Arg Thr His Val Ala Val Glu Arg Tyr Pro
                85                  90                  95

Thr Trp Gly Asp Thr Val Glu Val Glu Cys Trp Ile Gly Ala Ser Gly
            100                 105                 110

Asn Asn Gly Met Arg Arg Asp Phe Leu Val Arg Asp Cys Lys Thr Gly
        115                 120                 125

Glu Ile Leu Thr Arg Cys Thr Ser Leu Ser Val Leu Met Asn Thr Arg
    130                 135                 140

Thr Arg Arg Leu Ser Thr Ile Pro Asp Glu Val Arg Gly Glu Ile Gly
145                 150                 155                 160

Pro Ala Phe Ile Asp Asn Val Ala Val Lys Asp Asp Glu Ile Lys Lys
                165                 170                 175

Leu Gln Lys Leu Asn Asp Ser Thr Ala Asp Tyr Ile Gln Gly Gly Leu
            180                 185                 190

Thr Pro Arg Trp Asn Asp Leu Asp Val Asn Gln His Val Asn Asn Leu
        195                 200                 205

Lys Tyr Val Ala Trp Val Phe Glu Thr Val Pro Asp Ser Ile Phe Glu
    210                 215                 220

Ser His His Ile Ser Ser Phe Thr Leu Glu Tyr Arg Arg Glu Cys Thr
225                 230                 235                 240

Arg Asp Ser Val Leu Arg Ser Leu Thr Thr Val Ser Gly Gly Ser Ser
                245                 250                 255

Glu Ala Gly Leu Val Cys Asp His Leu Leu Gln Leu Glu Gly Gly Ser
            260                 265                 270

Glu Val Leu Arg Ala Arg Thr Glu Trp Arg Pro Lys Leu Thr Asp Ser
        275                 280                 285

Phe Arg Gly Ile Ser Val Ile Pro Ala Glu Pro Arg Val
    290                 295                 300

<210>  5
<211>  57
<212>  DNA
<213>  Escherichia coli

<400>  5
tagtcaaaag cctccggtcg gaggcttttg actttctgct tactgaattt gcggccg          57

<210>  6
<211>  1004
<212>  DNA
<213>  Artificial Sequence

<220>
<223>  codon optimised gene

<400>  6
ggtacctttc aggagattca agaatgcatg atcggaaatc caaacgcccc gatatgctcg       60

tggactcgtt cggcctggaa agcaccgtcc aggatgggct ggtgtttcgg caatcgtttt      120

cgatccggag ctatgaaatt ggcaccgacc ggacggcgag cattgaaacg ctgatgaacc      180

atctgcagga aacctccctg aaccactgca aatccaccgg cattctcctc gatgggtttg      240

ggcgcacgct cgaaatgtgt aagcgcgatc tgatctgggt ggtgattaag atgcagatta      300

aagtgaaccg gtatcccgcg tggggcgaca ccgtggagat caatacccgg ttttcgcgcc      360

tcgggaagat tgggatgggg cgggactggc tgattagcga ctgcaacacg ggcgagattc      420

tcgtccgggc cacctcggcc tatgccatga tgaatcagaa aacccgccgg ctgagcaaac      480

tgccctacga agtgcaccag gaaatcgtcc cgctcttcgt cgactccccg gtgatcgaag 540

attcggacct gaaggtgcat aagttcaagg tgaagacggg ggatagcatt cagaagggcc 600

tgacgccggg ctggaacgat ctcgacgtga accagcatgt gtccaacgtc aaatacatcg 660

gctggattct ggaatcgatg ccgacggaag tcctggagac ccaagaactg tgctccctcg 720

ccctcgagta tcgccgggag tgtggccgcg actcggtcct cgaatcggtg accgccatgg 780

acccgtcgaa ggtcggggtg cggtcgcaat atcaacacct gctgcgcctg gaggacggga 840

ccgcgattgt gaacggcgcc accgagtggc ggccgaaaaa tgcgggggcc aacggggcca 900

ttagcacggg caagacgagc aacggcaact ccgtgtcgta atagtcaaaa gcctccggtc 960

ggaggctttt gactttctgc ttactgaatt tgcggccgaa gctt 1004

<210> 7
<211> 1070
<212> DNA
<213> Artificial Sequence

<220>
<223> codon optimised gene

<400> 7
ggtacctttc aggagattca agaatgccgg actggtcgat gctgctggcg gccatccgca 60

ccattttcag cgccgcggag aagcagtgga ccctgctgga ctcgaagaag cggggggcgg 120

acgcggtggc cgacgcctcc ggggtgggca agatggtgaa gaatggcctg gtgtaccgcc 180

agaacttctc gatccgctcg tatgagatcg gggtggacaa gcgcgcctcg gtcgaggccc 240

tgatgaacca cttccaggag acctcgctca accactgcaa gtgcatcggc ctgatgcatg 300

gcggcttcgg ctgcacgccg gagatgacgc ggcgcaacct gatctgggtc gtggcgaaga 360

tgctggtgca cgtggagcgc tatccgtggt ggggcgacgt ggtgcagatt aacacctgga 420

tctcgtcgtc cgggaagaac ggcatgggcc gggattggca tgtgcacgat tgccaaaccg 480

gcctgccgat catgcgcggg acgtcggtgt gggtgatgat ggacaagcac acccggcggc 540

tctcgaagct gccggaggag gtgcgcgcgg aaatcacccc gtttttctcg gagcgcgatg 600

cggtgctcga tgacaacggc cgcaagctgc cgaagttcga cgatgactcc gccgcgcatg 660

tgcggcgcgg gctgaccccg cgctggcatg attttgacgt gaatcagcat gtgaacaacg 720

tgaagtacgt gggctggatt ctggagtccg tgcccgtgtg gatgctcgac gggtacgagg 780

tggccacgat gtcgctggag tatcgccgcg agtgccgcat ggactccgtg gtgcaatcgc 840

tgaccgccgt gtcgtcggac cacgccgacg gctcgccgat cgtgtgccag cacctgctgc 900

gcctggagga cggcaccgaa attgtgcggg gccagacgga gtggcgcccg aaacagcagg 960

cccgcgacct gggcaacatg ggctgcaccc cgacggagtc gaagtaatag tcaaaagcct 1020

ccggtcggag gcttttgact ttctgcttac tgaatttgcg gccgaagctt 1070

<210> 8
<211> 992
<212> DNA
<213> Artificial Sequence

<220>
<223> codon optimised gene

<400> 8
ggtacctttc aggagattca agaatgaccc tcgaatggaa gccgaaaccg aagctgccgc 60

agctcctgga cgatcatttt ggcctgcacg ggctggtgtt ccgccgcacg ttcgccatcc 120

gctcgtatga agtcggcccc gaccggagca cgtccatcct ggcggtgatg aatcacatgc 180

aagaggccac cctgaaccat gccaaatccg tgggcatcct cggcgacggc tttggcacca 240

cgctggagat gtcgaaacgc gacctgatgt gggtggtccg ccggacccat gtggccgtgg 300

agcgctaccc gacctggggc gacaccgtgg aagtggagtg ctggatcggc gcctcgggca 360

acaacggcat gcgccgggat ttcctggtgc gcgactgcaa gaccggcgag atcctcacgc 420

gctgcacctc gctgtcggtg ctcatgaata cccgcacgcg ccgcctgtcg accattccgg 480

acgaagtccg cggggagatt ggcccggcgt tcattgataa tgtcgcggtg aaggacgacg 540

agatcaagaa actccagaag ctgaacgact cgaccgccga ctacatccaa gggggcctga 600

cgccccggtg gaacgatctc gatgtgaacc agcacgtgaa caacctcaaa tatgtggcgt 660

gggtgtttga gaccgtgccg gactcgatct tcgaatcgca tcacatctcg agctttaccc 720

tggagtatcg ccgcgagtgc acccgggaca gcgtcctgcg ctcgctcacc accgtcagcg 780

gcgggtcgtc ggaagcgggg ctcgtgtgcg accacctcct gcagctggag ggggggtccg 840

aagtgctgcg cgcccggacc gagtggcggc ccaaactgac cgattccttc cgcggcattt 900

cggtcatccc cgcggagccg cgggtgtaat agtcaaaagc ctccggtcgg aggcttttga 960

ctttctgctt actgaatttg cggccgaagc tt 992

<210> 9
<211> 5144
<212> DNA
<213> Artificial Sequence

<220>
<223> vector

<400> 9
ctcgggccgt ctcttgggct tgatcggcct tcttgcgcat ctcacgcgct cctgcggcgg 60

cctgtagggc aggctcatac ccctgccgaa ccgcttttgt cagccggtcg gccacggctt 120

ccggcgtctc aacgcgcttt gagattccca gcttttcggc caatccctgc ggtgcatagg 180

cgcgtggctc gaccgcttgc gggctgatgg tgacgtggcc cactggtggc cgctccaggg 240

cctcgtagaa cgcctgaatg cgcgtgtgac gtgccttgct gccctcgatg ccccgttgca 300

gccctagatc ggccacagcg gccgcaaacg tggtctggtc gcgggtcatc tgcgctttgt 360

tgccgatgaa ctccttggcc gacagcctgc cgtcctgcgt cagcggcacc acgaacgcgg 420

tcatgtgcgg gctggtttcg tcacggtgga tgctggccgt cacgatgcga tccgccccgt 480

acttgtccgc cagccacttg tgcgccttct cgaagaacgc cgcctgctgt tcttggctgg 540

ccgacttcca ccattccggg ctggccgtca tgacgtactc gaccgccaac acagcgtcct 600

tgcgccgctt ctctggcagc aactcgcgca gtcggcccat cgcttcatcg gtgctgctgg 660

ccgcccagtg ctcgttctct ggcgtcctgc tggcgtcagc gttgggcgtc tcgcgctcgc 720

ggtaggcgtg cttgagactg gccgccacgt tgcccatttt cgccagcttc ttgcatcgca 780

tgatcgcgta tgccgccatg cctgcccctc ccttttggtg tccaaccggc tcgacggggg 840

cagcgcaagg cggtgcctcc ggcgggccac tcaatgcttg agtatactca ctagactttg 900

cttcgcaaag tcgtgaccgc ctacggcggc tgcggcgccc tacgggcttg ctctccgggc 960

ttcgccctgc gcggtcgctg cgctcccttg ccagcccgtg gatatgtgga cgatggccgc 1020

gagcggccac cggctggctc gcttcgctcg gcccgtggac aaccctgctg gacaagctga 1080

tggacaggct gcgcctgccc acgagcttga ccacagggat tgcccaccgg ctacccagcc 1140

ttcgaccaca tacccaccgg ctccaactgc gcggcctgcg gccttgcccc atcaattttt 1200

ttaatttctt ctggggaaaa gcctccggcc tgcggcctgc gcgcttcgct tgccggttgg 1260

acaccaagtg gaaggcgggt caaggctcgc gcagcgaccg cgcagcggct tggccttgac 1320

gcgcctggaa cgacccaagc ctatgcgagt gggggcagtc gaaggcgaag cccgcccgcc 1380

tgccccccga gcctcacggc ggcgagtgcg ggggttccaa gggggcagcg ccaccttggg 1440

caaggccgaa ggccgcgcag tcgatcaaca agccccggag gggccacttt ttgccggagg 1500

gggagccgcg ccgaaggcgt gggggaaccc cgcaggggtg cccttctttg ggcaccaaag 1560

aactagatat agggcgaaat gcgaaagact taaaaatcaa caacttaaaa aaggggggta 1620

cgcaacagct cattgcggca ccccccgcaa tagctcattg cgtaggttaa agaaaatctg 1680

taattgactg ccacttttac gcaacgcata attgttgtcg cgctgccgaa aagttgcagc 1740

tgattgcgca tggtgccgca accgtgcggc accctaccgc atggagataa gcatggccac 1800

gcagtccaga gaaatcggca ttcaagccaa gaacaagccc ggtcactggg tgcaaacgga 1860

acgcaaagcg catgaggcgt gggccgggct tattgcgagg aaacccacgg cggcaatgct 1920

gctgcatcac ctcgtggcgc agatgggcca ccagaacgcc gtggtggtca gccagaagac 1980

actttccaag ctcatcggac gttctttgcg gacggtccaa tacgcagtca aggacttggt 2040

ggccgagcgc tggatctccg tcgtgaagct caacggcccc ggcaccgtgt cggcctacgt 2100

ggtcaatgac cgcgtggcgt ggggccagcc ccgcgaccag ttgcgcctgt cggtgttcag 2160

tgccgccgtg gtggttgatc acgacgacca ggacgaatcg ctgttggggc atggcgacct 2220

gcgccgcatc ccgaccctgt atccgggcga gcagcaacta ccgaccggcc ccggcgagga 2280

gccgcccagc cagcccggca ttccgggcat ggaaccagac ctgccagcct tgaccgaaac 2340

ggaggaatgg gaacggcgcg ggcagcagcg cctgccgatg cccgatgagc cgtgttttct 2400

ggacgatggc gagccgttgg agccgccgac acgggtcacg ctgccgcgcc ggtagcactt 2460

gggttgcgca gcaacccgta agtgcgctgt tccagactat cggctgtagc cgcctcgccg 2520

ccctatacct tgtctgcctc cccgcgttgc gtcgcggtgc atggagccgg gccacctcga 2580

cctgaatgga agccggcggc acctcgctaa cggattcacc gtttttatca ggctctggga 2640

ggcagaataa atgatcatat cgtcaattat tacctccacg gggagagcct gagcaaactg 2700

gcctcaggca tttgagaagc acacggtcac actgcttccg gtagtcaata aaccggtaaa 2760

ccagcaatag acataagcgg ctatttaacg accctgccct gaaccgacga ccgggtcgaa 2820

tttgctttcg aatttctgcc attcatccgc ttattatcac ttattcaggc gtagcaccag 2880

gcgtttaagg gcaccaataa ctgccttaaa aaaattacgc cccgccctgc cactcatcgc 2940

agtcggccta ttggttaaaa aatgagctga tttaacaaaa atttaacgcg aattttaaca 3000

aaatattaac gcttacaatt tccattcgcc attcaggctg cgcaactgtt gggaagggcg 3060

atcggtgcgg gcctcttcgc tattacgcca gctggcgaaa gggggatgtg ctgcaaggcg 3120

attaagttgg gtaacgccag ggttttccca gtcacgacgt tgtaaaacga cggccagtga 3180

gcgcgcgtaa tacgactcac tatagggcga attggagctc caccgcggtg gcggccgctc 3240

tagaactagt ggatcccccg ggctgcagga attcgatatc aagcttatcg ataccgtcga 3300

cctcgagggg gggcccggta cccagctttt gttcccttta gtgagggtta attgcgcgct 3360

tggcgtaatc atggtcatag ctgtttcctg tgtgaaattg ttatccgctc acaattccac 3420

acaacatacg agccggaagc ataaagtgta aagcctgggg tgcctaatga gtgagctaac 3480

tcacattaat tgcgttgcgc tcactgcccg ctttccagtc gggaaacctg tcgtgccagc 3540

tgcattaatg aatcggccaa cgcgcgggga gaggcggttt gcgtattggg cgcatgcata 3600

aaaactgttg taattcatta agcattctgc cgacatggaa gccatcacaa acggcatgat 3660

gaacctgaat cgccagcggc atcagcacct tgtcgccttg cgtataatat ttgcccatgg 3720

gggtgggcga agaactccag catgagatcc ccgcgctgga ggatcatcca gccggcgtcc 3780

cggaaaacga ttccgaagcc caacctttca tagaaggcgg cggtggaatc gaaatctcgt 3840

gatggcaggt tgggcgtcgc ttggtcggtc atttcgaacc ccagagtccc gctcagaaga 3900

actcgtcaag aaggcgatag aaggcgatgc gctgcgaatc gggagcggcg ataccgtaaa 3960

gcacgaggaa gcggtcagcc cattcgccgc caagctcttc agcaatatca cgggtagcca 4020

acgctatgtc ctgatagcgg tccgccacac ccagccggcc acagtcgatg aatccagaaa 4080

agcggccatt ttccaccatg atattcggca agcaggcatc gccatgggtc acgacgagat 4140

cctcgccgtc gggcatgcgc gccttgagcc tggcgaacag ttcggctggc gcgagcccct 4200

gatgctcttc gtccagatca tcctgatcga caagaccggc ttccatccga gtacgtgctc 4260

gctcgatgcg atgtttcgct tggtggtcga atgggcaggt agccggatca agcgtatgca 4320

gccgccgcat tgcatcagcc atgatggata ctttctcggc aggagcaagg tgagatgaca 4380

ggagatcctg ccccggcact tcgcccaata gcagccagtc ccttcccgct tcagtgacaa 4440

cgtcgagcac agctgcgcaa ggaacgcccg tcgtggccag ccacgatagc cgcgctgcct 4500

cgtcctgcag ttcattcagg gcaccggaca ggtcggtctt gacaaaaaga accgggcgcc 4560

cctgcgctga cagccggaac acggcggcat cagagcagcc gattgtctgt tgtgcccagt 4620

catagccgaa tagcctctcc acccaagcgg ccggagaacc tgcgtgcaat ccatcttgtt 4680

caatcatgcg aaacgatcct catcctgtct cttgatcaga tcttgatccc ctgcgccatc 4740

agatccttgg cggcaagaaa gccatccagt ttactttgca gggcttccca accttaccag 4800

agggcgcccc agctggcaat tccggttcgc ttgctgtcca taaaaccgcc cagtctagct 4860

atcgccatgt aagcccactg caagctacct gctttctctt tgcgcttgcg ttttcccttg 4920

tccagatagc ccagtagctg acattcatcc caggtggcac ttttcgggga aatgtgcgcg 4980

cccgcgttcc tgctggcgct gggcctgttt ctggcgctgg acttcccgct gttccgtcag 5040

cagcttttcg cccacggcct tgatgatcgc ggcggccttg gcctgcatat cccgattcaa 5100

cggccccagg gcgtccagaa cgggcttcag gcgctcccga aggt 5144

<210> 10
<211> 6106
<212> DNA
<213> Artificial Sequence

<220>
<223> vector

<400> 10
ctcgggccgt ctcttgggct tgatcggcct tcttgcgcat ctcacgcgct cctgcggcgg 60

cctgtagggc aggctcatac ccctgccgaa ccgcttttgt cagccggtcg gccacggctt 120

ccggcgtctc aacgcgcttt gagattccca gcttttcggc caatccctgc ggtgcatagg 180

cgcgtggctc gaccgcttgc gggctgatgg tgacgtggcc cactggtggc cgctccaggg 240

cctcgtagaa cgcctgaatg cgcgtgtgac gtgccttgct gccctcgatg ccccgttgca 300

gccctagatc ggccacagcg gccgcaaacg tggtctggtc gcgggtcatc tgcgctttgt 360

tgccgatgaa ctccttggcc gacagcctgc cgtcctgcgt cagcggcacc acgaacgcgg 420

tcatgtgcgg gctggtttcg tcacggtgga tgctggccgt cacgatgcga tccgccccgt 480

acttgtccgc cagccacttg tgcgccttct cgaagaacgc cgcctgctgt tcttggctgg 540

ccgacttcca ccattccggg ctggccgtca tgacgtactc gaccgccaac acagcgtcct 600

tgcgccgctt ctctggcagc aactcgcgca gtcggcccat cgcttcatcg gtgctgctgg 660

ccgcccagtg ctcgttctct ggcgtcctgc tggcgtcagc gttgggcgtc tcgcgctcgc 720

ggtaggcgtg cttgagactg gccgccacgt tgcccatttt cgccagcttc ttgcatcgca 780

tgatcgcgta tgccgccatg cctgcccctc ccttttggtg tccaaccggc tcgacggggg 840

cagcgcaagg cggtgcctcc ggcgggccac tcaatgcttg agtatactca ctagactttg 900

cttcgcaaag tcgtgaccgc ctacggcggc tgcggcgccc tacgggcttg ctctccgggc 960

ttcgccctgc gcggtcgctg cgctcccttg ccagcccgtg gatatgtgga cgatggccgc 1020

gagcggccac cggctggctc gcttcgctcg gcccgtggac aaccctgctg gacaagctga 1080

tggacaggct gcgcctgccc acgagcttga ccacagggat tgcccaccgg ctacccagcc 1140

ttcgaccaca tacccaccgg ctccaactgc gcggcctgcg gccttgcccc atcaattttt 1200

ttaattttct ctggggaaaa gcctccggcc tgcggcctgc gcgcttcgct tgccggttgg 1260

acaccaagtg gaaggcgggt caaggctcgc gcagcgaccg cgcagcggct tggccttgac 1320

gcgcctggaa cgacccaagc ctatgcgagt gggggcagtc gaaggcgaag cccgcccgcc 1380

tgccccccga gcctcacggc ggcgagtgcg ggggttccaa gggggcagcg ccaccttggg 1440

caaggccgaa ggccgcgcag tcgatcaaca agccccggag gggccacttt ttgccggagg 1500

gggagccgcg ccgaaggcgt gggggaaccc cgcaggggtg cccttctttg ggcaccaaag 1560

aactagatat agggcgaaat gcgaaagact taaaaatcaa caacttaaaa aaggggggta 1620

cgcaacagct cattgcggca ccccccgcaa tagctcattg cgtaggttaa agaaaatctg 1680

taattgactg ccacttttac gcaacgcata attgttgtcg cgctgccgaa aagttgcagc 1740

tgattgcgca tggtgccgca accgtgcggc accctaccgc atggagataa gcatggccac 1800

gcagtccaga gaaatcggca ttcaagccaa gaacaagccc ggtcactggg tgcaaacgga 1860

acgcaaagcg catgaggcgt gggccgggct tattgcgagg aaacccacgg cggcaatgct 1920

gctgcatcac ctcgtggcgc agatgggcca ccagaacgcc gtggtggtca gccagaagac 1980

actttccaag ctcatcggac gttctttgcg gacggtccaa tacgcagtca aggacttggt 2040

ggccgagcgc tggatctccg tcgtgaagct caacggcccc ggcaccgtgt cggcctacgt 2100

ggtcaatgac cgcgtggcgt ggggccagcc ccgcgaccag ttgcgcctgt cggtgttcag 2160

tgccgccgtg gtggttgatc acgacgacca ggacgaatcg ctgttggggc atggcgacct 2220

gcgccgcatc ccgaccctgt atccgggcga gcagcaacta ccgaccggcc ccggcgagga 2280

gccgcccagc cagcccggca ttccgggcat ggaaccagac ctgccagcct tgaccgaaac 2340

ggaggaatgg gaacggcgcg ggcagcagcg cctgccgatg cccgatgagc cgtgttttct 2400
ggacgatggc gagccgttgg agccgccgac acgggtcacg ctgccgcgcc ggtagcactt 2460
gggttgcgca gcaacccgta agtgcgctgt tccagactat cggctgtagc cgcctcgccg 2520
ccctatacct tgtctgcctc cccgcgttgc gtcgcggtgc atggagccgg gccacctcga 2580
cctgaatgga agccggcggc acctcgctaa cggattcacc gtttttatca ggctctggga 2640
ggcagaataa atgatcatat cgtcaattat tacctccacg gggagagcct gagcaaactg 2700
gcctcaggca tttgagaagc acacggtcac actgcttccg gtagtcaata aaccggtaaa 2760
ccagcaatag acataagcgg ctatttaacg accctgccct gaaccgacga ccgggtcgaa 2820
tttgctttcg aatttctgcc attcatccgc ttattatcac ttattcaggc gtagcaccag 2880
gcgtttaagg gcaccaataa ctgccttaaa aaaattacgc cccgccctgc cactcatcgc 2940
agtcggccta ttggttaaaa aatgagctga tttaacaaaa atttaacgcg aattttaaca 3000
aaatattaac gcttacaatt tccattcgcc attcaggctg cgcaactgtt gggaagggcg 3060
atcggtgcgg gcctcttcgc tattacgcca gctggcgaaa gggggatgtg ctgcaaggcg 3120
attaagttgg gtaacgccag ggttttccca gtcacgacgt tgtaaaacga cggccagtga 3180
gcgcgcgtaa tacgactcac tatagggcga attggagctc caccgcggtg gcggccgctc 3240
tagaactagt ggatcccccg ggctgcagga attcgatatc aagcttcggc cgcaaattca 3300
gtaagcagaa agtcaaaagc ctccgaccgg aggcttttga ctattacgac acggagttgc 3360
cgttgctcgt cttgcccgtg ctaatggccc cgttggcccc cgcatttttc ggccgccact 3420
cggtggcgcc gttcacaatc gcggtcccgt cctccaggcg cagcaggtgt tgatattgcg 3480
accgcacccc gaccttcgac gggtccatgg cggtcaccga ttcgaggacc gagtcgcggc 3540
cacactcccg gcgatactcg agggcgaggg agcacagttc ttgggtctcc aggacttccg 3600
tcggcatcga ttccagaatc cagccgatgt atttgacgtt ggacacatgc tggttcacgt 3660
cgagatcgtt ccagcccggc gtcaggccct tctgaatgct atcccccgtc ttcaccttga 3720
acttatgcac cttcaggtcc gaatcttcga tcaccgggga gtcgacgaag agcgggacga 3780
tttcctggtg cacttcgtag ggcagtttgc tcagccggcg ggttttctga ttcatcatgg 3840
cataggccga ggtggcccgg acgagaatct cgcccgtgtt gcagtcgcta atcagccagt 3900
cccgccccat cccaatcttc ccgaggcgcg aaaaccgggt attgatctcc acggtgtcgc 3960
cccacgcggg ataccggttc actttaatct gcatcttaat caccacccag atcagatcgc 4020
gcttacacat ttcgagcgtg cgcccaaacc catcgaggag aatgccggtg gatttgcagt 4080
ggttcaggga ggtttcctgc agatggttca tcagcgtttc aatgctcgcc gtccggtcgg 4140
tgccaatttc atagctccgg atcgaaaacg attgccgaaa caccagccca tcctggacgg 4200

tgctttccag gccgaacgag tccacgagca tatcggggcg tttggatttc cgatcatgca 4260

ttcttgaatc tcctgaaagg tacccagctt ttgttccctt tagtgagggt taattgcgcg 4320

cttggcgtaa tcatggtcat agctgtttcc tgtgtgaaat tgttatccgc tcacaattcc 4380

acacaacata cgagccggaa gcataaagtg taaagcctgg ggtgcctaat gagtgagcta 4440

actcacatta attgcgttgc gctcactgcc cgctttccag tcgggaaacc tgtcgtgcca 4500

gctgcattaa tgaatcggcc aacgcgcggg gagaggcggt ttgcgtattg ggcgcatgca 4560

taaaaactgt tgtaattcat taagcattct gccgacatgg aagccatcac aaacggcatg 4620

atgaacctga atcgccagcg gcatcagcac cttgtcgcct tgcgtataat atttgcccat 4680

gggggtgggc gaagaactcc agcatgagat ccccgcgctg gaggatcatc cagccggcgt 4740

cccggaaaac gattccgaag cccaaccttt catagaaggc ggcggtggaa tcgaaatctc 4800

gtgatggcag gttgggcgtc gcttggtcgg tcatttcgaa ccccagagtc ccgctcagaa 4860

gaactcgtca agaaggcgat agaaggcgat gcgctgcgaa tcgggagcgg cgataccgta 4920

aagcacgagg aagcggtcag cccattcgcc gccaagctct tcagcaatat cacgggtagc 4980

caacgctatg tcctgatagc ggtccgccac acccagccgg ccacagtcga tgaatccaga 5040

aaagcggcca ttttccacca tgatattcgg caagcaggca tcgccatggg tcacgacgag 5100

atcctcgccg tcgggcatgc gcgccttgag cctggcgaac agttcggctg gcgcgagccc 5160

ctgatgctct tcgtccagat catcctgatc gacaagaccg gcttccatcc gagtacgtgc 5220

tcgctcgatg cgatgtttcg cttggtggtc gaatgggcag gtagccggat caagcgtatg 5280

cagccgccgc attgcatcag ccatgatgga tactttctcg gcaggagcaa ggtgagatga 5340

caggagatcc tgccccggca cttcgcccaa tagcagccag tcccttcccg cttcagtgac 5400

aacgtcgagc acagctgcgc aaggaacgcc cgtcgtggcc agccacgata gccgcgctgc 5460

ctcgtcctgc agttcattca gggcaccgga caggtcggtc ttgacaaaaa gaaccgggcg 5520

cccctgcgct gacagccgga acacggcggc atcagagcag ccgattgtct gttgtgccca 5580

gtcatagccg aatagcctct ccacccaagc ggccggagaa cctgcgtgca atccatcttg 5640

ttcaatcatg cgaaacgatc ctcatcctgt ctcttgatca gatcttgatc ccctgcgcca 5700

tcagatcctt ggcggcaaga aagccatcca gtttactttg cagggcttcc caaccttacc 5760

agagggcgcc ccagctggca attccggttc gcttgctgtc cataaaaccg cccagtctag 5820

ctatcgccat gtaagcccac tgcaagctac ctgctttctc tttgcgcttg cgttttccct 5880

tgtccagata gcccagtagc tgacattcat cccaggtggc acttttcggg gaaatgtgcg 5940

cgcccgcgtt cctgctggcg ctgggcctgt ttctggcgct ggacttcccg ctgttccgtc 6000

agcagctttt cgcccacggc cttgatgatc gcggcggcct tggcctgcat atcccgattc 6060

aacggcccca gggcgtccag aacgggcttc aggcgctccc gaaggt 6106

<210>  11
<211>  6172
<212>  DNA
<213>  Artificial Sequence

<220>
<223>  vector

<400>  11
ctcgggccgt ctcttgggct tgatcggcct tcttgcgcat ctcacgcgct cctgcggcgg      60

cctgtagggc aggctcatac ccctgccgaa ccgcttttgt cagccggtcg gccacggctt     120

ccggcgtctc aacgcgcttt gagattccca gcttttcggc caatccctgc ggtgcatagg     180

cgcgtggctc gaccgcttgc gggctgatgg tgacgtggcc cactggtggc cgctccaggg     240

cctcgtagaa cgcctgaatg cgcgtgtgac gtgccttgct gccctcgatg ccccgttgca     300

gccctagatc ggccacagcg gccgcaaacg tggtctggtc gcgggtcatc tgcgctttgt     360

tgccgatgaa ctccttggcc gacagcctgc cgtcctgcgt cagcggcacc acgaacgcgg     420

tcatgtgcgg gctggtttcg tcacggtgga tgctggccgt cacgatgcga tccgccccgt     480

acttgtccgc cagccacttg tgcgccttct cgaagaacgc cgcctgctgt tcttggctgg     540

ccgacttcca ccattccggg ctggccgtca tgacgtactc gaccgccaac acagcgtcct     600

tgcgccgctt ctctggcagc aactcgcgca gtcggcccat cgcttcatcg gtgctgctgg     660

ccgcccagtg ctcgttctct ggcgtcctgc tggcgtcagc gttgggcgtc tcgcgctcgc     720

ggtaggcgtg cttgagactg gccgccacgt tgcccatttt cgccagcttc ttgcatcgca     780

tgatcgcgta tgccgccatg cctgcccctc ccttttggtg tccaaccggc tcgacggggg     840

cagcgcaagg cggtgcctcc ggcgggccac tcaatgcttg agtatactca ctagactttg     900

cttcgcaaag tcgtgaccgc ctacggcggc tgcggcgccc tacgggcttg ctctccgggc     960

ttcgccctgc gcggtcgctg cgctcccttg ccagcccgtg gatatgtgga cgatggccgc    1020

gagcggccac cggctggctc gcttcgctcg gcccgtggac aaccctgctg gacaagctga    1080

tggacaggct gcgcctgccc acgagcttga ccacagggat tgcccaccgg ctacccagcc    1140

ttcgaccaca tacccaccgg ctccaactgc gcggcctgcg gccttgcccc atcaattttt    1200

ttaattttct ctggggaaaa gcctccggcc tgcggcctgc gcgcttcgct tgccggttgg    1260

acaccaagtg gaaggcgggt caaggctcgc gcagcgaccg cgcagcggct tggccttgac    1320

gcgcctggaa cgacccaagc ctatgcgagt gggggcagtc gaaggcgaag cccgcccgcc    1380

tgccccccga gcctcacggc ggcgagtgcg ggggttccaa gggggcagcg ccaccttggg    1440

caaggccgaa ggccgcgcag tcgatcaaca agccccggag gggccacttt ttgccggagg    1500

gggagccgcg ccgaaggcgt gggggaaccc cgcaggggtg cccttctttg ggcaccaaag    1560

aactagatat agggcgaaat gcgaaagact taaaaatcaa caacttaaaa aaggggggta 1620

cgcaacagct cattgcggca ccccccgcaa tagctcattg cgtaggttaa agaaaatctg 1680

taattgactg ccacttttac gcaacgcata attgttgtcg cgctgccgaa aagttgcagc 1740

tgattgcgca tggtgccgca accgtgcggc accctaccgc atggagataa gcatggccac 1800

gcagtccaga gaaatcggca ttcaagccaa gaacaagccc ggtcactggg tgcaaacgga 1860

acgcaaagcg catgaggcgt gggccgggct tattgcgagg aaacccacgg cggcaatgct 1920

gctgcatcac ctcgtggcgc agatgggcca ccagaacgcc gtggtggtca gccagaagac 1980

actttccaag ctcatcggac gttctttgcg gacggtccaa tacgcagtca aggacttggt 2040

ggccgagcgc tggatctccg tcgtgaagct caacggcccc ggcaccgtgt cggcctacgt 2100

ggtcaatgac cgcgtggcgt ggggccagcc ccgcgaccag ttgcgcctgt cggtgttcag 2160

tgccgccgtg gtggttgatc acgacgacca ggacgaatcg ctgttggggc atggcgacct 2220

gcgccgcatc ccgaccctgt atccgggcga gcagcaacta ccgaccggcc ccggcgagga 2280

gccgcccagc cagcccggca ttccgggcat ggaaccagac ctgccagcct tgaccgaaac 2340

ggaggaatgg gaacggcgcg ggcagcagcg cctgccgatg cccgatgagc cgtgttttct 2400

ggacgatggc gagccgttgg agccgccgac acgggtcacg ctgccgcgcc ggtagcactt 2460

gggttgcgca gcaacccgta agtgcgctgt tccagactat cggctgtagc cgcctcgccg 2520

ccctatacct tgtctgcctc cccgcgttgc gtcgcggtgc atggagccgg gccacctcga 2580

cctgaatgga agccggcggc acctcgctaa cggattcacc gtttttatca ggctctggga 2640

ggcagaataa atgatcatat cgtcaattat tacctccacg gggagagcct gagcaaactg 2700

gcctcaggca tttgagaagc acacggtcac actgcttccg gtagtcaata aaccggtaaa 2760

ccagcaatag acataagcgg ctatttaacg accctgccct gaaccgacga ccgggtcgaa 2820

tttgctttcg aatttctgcc attcatccgc ttattatcac ttattcaggc gtagcaccag 2880

gcgtttaagg gcaccaataa ctgccttaaa aaaattacgc cccgccctgc cactcatcgc 2940

agtcggccta ttggttaaaa aatgagctga tttaacaaaa atttaacgcg aattttaaca 3000

aaatattaac gcttacaatt tccattcgcc attcaggctg cgcaactgtt gggaagggcg 3060

atcggtgcgg gcctcttcgc tattacgcca gctggcgaaa gggggatgtg ctgcaaggcg 3120

attaagttgg gtaacgccag ggttttccca gtcacgacgt tgtaaaacga cggccagtga 3180

gcgcgcgtaa tacgactcac tatagggcga attggagctc caccgcggtg gcggccgctc 3240

tagaactagt ggatcccccg ggctgcagga attcgatatc aagcttcggc cgcaaattca 3300

gtaagcagaa agtcaaaagc ctccgaccgg aggcttttga ctattacttc gactccgtcg 3360

ggtgcagccc catgttgccc aggtcgcggg cctgctgttt cgggcgccac tccgtctggc 3420

cccgcacaat ttcggtgccg tcctccaggc gcagcaggtg ctggcacacg atcggcgagc 3480

cgtcggcgtg gtccgacgac acggcggtca gcgattgcac cacggagtcc atgcggcact 3540

cgcggcgata ctccagcgac atcgtggcca cctcgtaccc gtcgagcatc cacacgggca 3600

cggactccag aatccagccc acgtacttca cgttgttcac atgctgattc acgtcaaaat 3660

catgccagcg cggggtcagc ccgcgccgca catgcgcggc ggagtcatcg tcgaacttcg 3720

gcagcttgcg gccgttgtca tcgagcaccg catcgcgctc cgagaaaaac ggggtgattt 3780

ccgcgcgcac ctcctccggc agcttcgaga gccgccgggt gtgcttgtcc atcatcaccc 3840

acaccgacgt cccgcgcatg atcggcaggc cggtttggca atcgtgcaca tgccaatccc 3900

ggcccatgcc gttcttcccg gacgacgaga tccaggtgtt aatctgcacc acgtcgcccc 3960

accacggata gcgctccacg tgcaccagca tcttcgccac gacccagatc aggttgcgcc 4020

gcgtcatctc cggcgtgcag ccgaagccgc catgcatcag gccgatgcac ttgcagtggt 4080

tgagcgaggt ctcctggaag tggttcatca gggcctcgac cgaggcgcgc ttgtccaccc 4140

cgatctcata cgagcggatc gagaagttct ggcggtacac caggccattc ttcaccatct 4200

tgcccacccc ggaggcgtcg gccaccgcgt ccgccccccg cttcttcgag tccagcaggg 4260

tccactgctt ctccgcggcg ctgaaaatgg tgcggatggc cgccagcagc atcgaccagt 4320

ccggcattct tgaatctcct gaaaggtacc cagcttttgt tccctttagt gagggttaat 4380

tgcgcgcttg gcgtaatcat ggtcatagct gtttcctgtg tgaaattgtt atccgctcac 4440

aattccacac aacatacgag ccggaagcat aaagtgtaaa gcctggggtg cctaatgagt 4500

gagctaactc acattaattg cgttgcgctc actgcccgct ttccagtcgg gaaacctgtc 4560

gtgccagctg cattaatgaa tcggccaacg cgcggggaga ggcggtttgc gtattgggcg 4620

catgcataaa aactgttgta attcattaag cattctgccg acatggaagc catcacaaac 4680

ggcatgatga acctgaatcg ccagcggcat cagcaccttg tcgccttgcg tataatattt 4740

gcccatgggg gtgggcgaag aactccagca tgagatcccc gcgctggagg atcatccagc 4800

cggcgtcccg gaaaacgatt ccgaagccca acctttcata gaaggcggcg gtggaatcga 4860

aatctcgtga tggcaggttg ggcgtcgctt ggtcggtcat ttcgaacccc agagtcccgc 4920

tcagaagaac tcgtcaagaa ggcgatagaa ggcgatgcgc tgcgaatcgg gagcggcgat 4980

accgtaaagc acgaggaagc ggtcagccca ttcgccgcca agctcttcag caatatcacg 5040

ggtagccaac gctatgtcct gatagcggtc cgccacaccc agccggccac agtcgatgaa 5100

tccagaaaag cggccatttt ccaccatgat attcggcaag caggcatcgc catgggtcac 5160

gacgagatcc tcgccgtcgg gcatgcgcgc cttgagcctg gcgaacagtt cggctggcgc 5220

gagcccctga tgctcttcgt ccagatcatc ctgatcgaca agaccggctt ccatccgagt 5280

acgtgctcgc tcgatgcgat gtttcgcttg gtggtcgaat gggcaggtag ccggatcaag 5340

cgtatgcagc cgccgcattg catcagccat gatggatact ttctcggcag gagcaaggtg 5400

agatgacagg agatcctgcc ccggcacttc gcccaatagc agccagtccc ttcccgcttc 5460

agtgacaacg tcgagcacag ctgcgcaagg aacgcccgtc gtggccagcc acgatagccg 5520

cgctgcctcg tcctgcagtt cattcagggc accggacagg tcggtcttga caaaaagaac 5580

cgggcgcccc tgcgctgaca gccggaacac ggcggcatca gagcagccga ttgtctgttg 5640

tgcccagtca tagccgaata gcctctccac ccaagcggcc ggagaacctg cgtgcaatcc 5700

atcttgttca atcatgcgaa acgatcctca tcctgtctct tgatcagatc ttgatcccct 5760

gcgccatcag atccttggcg gcaagaaagc catccagttt actttgcagg gcttcccaac 5820

cttaccagag ggcgccccag ctggcaattc cggttcgctt gctgtccata aaaccgccca 5880

gtctagctat cgccatgtaa gcccactgca agctacctgc tttctctttg cgcttgcgtt 5940

ttcccttgtc cagatagccc agtagctgac attcatccca ggtggcactt ttcggggaaa 6000

tgtgcgcgcc cgcgttcctg ctggcgctgg gcctgtttct ggcgctggac ttcccgctgt 6060

tccgtcagca gcttttcgcc cacggccttg atgatcgcgg cggccttggc ctgcatatcc 6120

cgattcaacg gccccagggc gtccagaacg ggcttcaggc gctcccgaag gt 6172

<210> 12
<211> 6094
<212> DNA
<213> Artificial Sequence

<220>
<223> vector

<400> 12
ctcgggccgt ctcttgggct tgatcggcct tcttgcgcat ctcacgcgct cctgcggcgg 60

cctgtagggc aggctcatac ccctgccgaa ccgcttttgt cagccggtcg gccacggctt 120

ccggcgtctc aacgcgcttt gagattccca gcttttcggc caatccctgc ggtgcatagg 180

cgcgtggctc gaccgcttgc gggctgatgg tgacgtggcc cactggtggc cgctccaggg 240

cctcgtagaa cgcctgaatg cgcgtgtgac gtgccttgct gccctcgatg ccccgttgca 300

gccctagatc ggccacagcg gccgcaaacg tggtctggtc gcgggtcatc tgcgctttgt 360

tgccgatgaa ctccttggcc gacagcctgc cgtcctgcgt cagcggcacc acgaacgcgg 420

tcatgtgcgg gctggtttcg tcacggtgga tgctggccgt cacgatgcga tccgccccgt 480

acttgtccgc cagccacttg tgcgccttct cgaagaacgc cgcctgctgt tcttggctgg 540

ccgacttcca ccattccggg ctggccgtca tgacgtactc gaccgccaac acagcgtcct 600

tgcgccgctt ctctggcagc aactcgcgca gtcggcccat cgcttcatcg gtgctgctgg 660

ccgcccagtg ctcgttctct ggcgtcctgc tggcgtcagc gttgggcgtc tcgcgctcgc 720

ggtaggcgtg cttgagactg gccgccacgt tgcccatttt cgccagcttc ttgcatcgca 780

tgatcgcgta tgccgccatg cctgcccctc ccttttggtg tccaaccggc tcgacggggg 840

cagcgcaagg cggtgcctcc ggcgggccac tcaatgcttg agtatactca ctagactttg 900

cttcgcaaag tcgtgaccgc ctacggcggc tgcggcgccc tacgggcttg ctctccgggc 960

ttcgccctgc gcggtcgctg cgctcccttg ccagcccgtg gatatgtgga cgatggccgc 1020

gagcggccac cggctggctc gcttcgctcg gcccgtggac aaccctgctg gacaagctga 1080

tggacaggct gcgcctgccc acgagcttga ccacagggat tgcccaccgg ctacccagcc 1140

ttcgaccaca tacccaccgg ctccaactgc gcggcctgcg gccttgcccc atcaattttt 1200

ttaattttct ctggggaaaa gcctccggcc tgcggcctgc gcgcttcgct tgccggttgg 1260

acaccaagtg gaaggcgggt caaggctcgc gcagcgaccg cgcagcggct tggccttgac 1320

gcgcctggaa cgacccaagc ctatgcgagt gggggcagtc gaaggcgaag cccgcccgcc 1380

tgccccccga gcctcacggc ggcgagtgcg ggggttccaa gggggcagcg ccaccttggg 1440

caaggccgaa ggccgcgcag tcgatcaaca agccccggag gggccacttt ttgccggagg 1500

gggagccgcg ccgaaggcgt gggggaaccc cgcaggggtg cccttctttg ggcaccaaag 1560

aactagatat agggcgaaat gcgaaagact taaaaatcaa caacttaaaa aaggggggta 1620

cgcaacagct cattgcggca ccccccgcaa tagctcattg cgtaggttaa agaaaatctg 1680

taattgactg ccacttttac gcaacgcata attgttgtcg cgctgccgaa aagttgcagc 1740

tgattgcgca tggtgccgca accgtgcggc accctaccgc atggagataa gcatggccac 1800

gcagtccaga gaaatcggca ttcaagccaa gaacaagccc ggtcactggg tgcaaacgga 1860

acgcaaagcg catgaggcgt gggccgggct tattgcgagg aaacccacgg cggcaatgct 1920

gctgcatcac ctcgtggcgc agatgggcca ccagaacgcc gtggtggtca gccagaagac 1980

actttccaag ctcatcggac gttctttgcg gacggtccaa tacgcagtca aggacttggt 2040

ggccgagcgc tggatctccg tcgtgaagct caacggcccc ggcaccgtgt cggcctacgt 2100

ggtcaatgac cgcgtggcgt ggggccagcc ccgcgaccag ttgcgcctgt cggtgttcag 2160

tgccgccgtg gtggttgatc acgacgacca ggacgaatcg ctgttggggc atggcgacct 2220

gcgccgcatc ccgaccctgt atccgggcga gcagcaacta ccgaccggcc ccggcgagga 2280

gccgcccagc cagcccggca ttccgggcat ggaaccagac ctgccagcct tgaccgaaac 2340

ggaggaatgg gaacggcgcg ggcagcagcg cctgccgatg cccgatgagc cgtgttttct 2400

ggacgatggc gagccgttgg agccgccgac acgggtcacg ctgccgcgcc ggtagcactt 2460

gggttgcgca gcaacccgta agtgcgctgt tccagactat cggctgtagc cgcctcgccg 2520

ccctatacct tgtctgcctc cccgcgttgc gtcgcggtgc atggagccgg gccacctcga 2580

cctgaatgga agccggcggc acctcgctaa cggattcacc gtttttatca ggctctggga 2640

ggcagaataa atgatcatat cgtcaattat tacctccacg gggagagcct gagcaaactg 2700

gcctcaggca tttgagaagc acacggtcac actgcttccg gtagtcaata aaccggtaaa 2760

ccagcaatag acataagcgg ctatttaacg accctgccct gaaccgacga ccgggtcgaa 2820

tttgctttcg aatttctgcc attcatccgc ttattatcac ttattcaggc gtagcaccag 2880

gcgtttaagg gcaccaataa ctgccttaaa aaaattacgc cccgccctgc cactcatcgc 2940

agtcggccta ttggttaaaa aatgagctga tttaacaaaa atttaacgcg aattttaaca 3000

aaatattaac gcttacaatt tccattcgcc attcaggctg cgcaactgtt gggaagggcg 3060

atcggtgcgg gcctcttcgc tattacgcca gctggcgaaa gggggatgtg ctgcaaggcg 3120

attaagttgg gtaacgccag ggttttccca gtcacgacgt tgtaaaacga cggccagtga 3180

gcgcgcgtaa tacgactcac tatagggcga attggagctc caccgcggtg gcggccgctc 3240

tagaactagt ggatcccccg ggctgcagga attcgatatc aagcttcggc cgcaaattca 3300

gtaagcagaa agtcaaaagc ctccgaccgg aggcttttga ctattacacc cgcggctccg 3360

cggggatgac cgaaatgccg cggaaggaat cggtcagttt gggccgccac tcggtccggg 3420

cgcgcagcac ttcggacccc ccctccagct gcaggaggtg gtcgcacacg agccccgctt 3480

ccgacgaccc gccgctgacg gtggtgagcg agcgcaggac gctgtcccgg gtgcactcgc 3540

ggcgatactc cagggtaaag ctcgagatgt gatgcgattc gaagatcgag tccggcacgg 3600

tctcaaacac ccacgccaca tatttgaggt tgttcacgtg ctggttcaca tcgagatcgt 3660

tccaccgggg cgtcaggccc ccttggatgt agtcggcggt cgagtcgttc agcttctgga 3720

gtttcttgat ctcgtcgtcc ttcaccgcga cattatcaat gaacgccggg ccaatctccc 3780

cgcggacttc gtccggaatg gtcgacaggc ggcgcgtgcg ggtattcatg agcaccgaca 3840

gcgaggtgca gcgcgtgagg atctcgccgg tcttgcagtc gcgcaccagg aaatcccggc 3900

gcatgccgtt gttgcccgag gcgccgatcc agcactccac ttccacggtg tcgccccagg 3960

tcgggtagcg ctccacggcc acatgggtcc ggcggaccac ccacatcagg tcgcgtttcg 4020

acatctccag cgtggtgcca aagccgtcgc cgaggatgcc cacggatttg gcatggttca 4080

gggtggcctc ttgcatgtga ttcatcaccg ccaggatgga cgtgctccgg tcggggccga 4140

cttcatacga gcggatggcg aacgtgcggc ggaacaccag cccgtgcagg ccaaaatgat 4200

cgtccaggag ctgcggcagc ttcggtttcg gcttccattc gagggtcatt cttgaatctc 4260

ctgaaaggta cccagctttt gttcccttta gtgagggtta attgcgcgct tggcgtaatc 4320

atggtcatag ctgtttcctg tgtgaaattg ttatccgctc acaattccac acaacatacg 4380

agccggaagc ataaagtgta aagcctgggg tgcctaatga gtgagctaac tcacattaat 4440

tgcgttgcgc tcactgcccg ctttccagtc gggaaacctg tcgtgccagc tgcattaatg 4500

aatcggccaa cgcgcgggga gaggcggttt gcgtattggg cgcatgcata aaaactgttg 4560

taattcatta agcattctgc cgacatggaa gccatcacaa acggcatgat gaacctgaat 4620

cgccagcggc atcagcacct tgtcgccttg cgtataatat ttgcccatgg gggtgggcga 4680

agaactccag catgagatcc ccgcgctgga ggatcatcca gccggcgtcc cggaaaacga 4740

ttccgaagcc caacctttca tagaaggcgg cggtggaatc gaaatctcgt gatggcaggt 4800

tgggcgtcgc ttggtcggtc atttcgaacc ccagagtccc gctcagaaga actcgtcaag 4860

aaggcgatag aaggcgatgc gctgcgaatc gggagcggcg ataccgtaaa gcacgaggaa 4920

gcggtcagcc cattcgccgc caagctcttc agcaatatca cgggtagcca acgctatgtc 4980

ctgatagcgg tccgccacac ccagccggcc acagtcgatg aatccagaaa agcggccatt 5040

ttccaccatg atattcggca agcaggcatc gccatgggtc acgacgagat cctcgccgtc 5100

gggcatgcgc gccttgagcc tggcgaacag ttcggctggc gcgagcccct gatgctcttc 5160

gtccagatca tcctgatcga caagaccggc ttccatccga gtacgtgctc gctcgatgcg 5220

atgtttcgct tggtggtcga atgggcaggt agccggatca agcgtatgca gccgccgcat 5280

tgcatcagcc atgatggata ctttctcggc aggagcaagg tgagatgaca ggagatcctg 5340

ccccggcact tcgcccaata gcagccagtc ccttcccgct tcagtgacaa cgtcgagcac 5400

agctgcgcaa ggaacgcccg tcgtggccag ccacgatagc cgcgctgcct cgtcctgcag 5460

ttcattcagg gcaccggaca ggtcggtctt gacaaaaaga accgggcgcc cctgcgctga 5520

cagccggaac acggcggcat cagagcagcc gattgtctgt tgtgcccagt catagccgaa 5580

tagcctctcc acccaagcgg ccggagaacc tgcgtgcaat ccatcttgtt caatcatgcg 5640

aaacgatcct catcctgtct cttgatcaga tcttgatccc ctgcgccatc agatccttgg 5700

cggcaagaaa gccatccagt ttactttgca gggcttccca accttaccag agggcgcccc 5760

agctggcaat tccggttcgc ttgctgtcca taaaaccgcc cagtctagct atcgccatgt 5820

aagcccactg caagctacct gctttctctt tgcgcttgcg ttttcccttg tccagatagc 5880

ccagtagctg acattcatcc caggtggcac ttttcgggga aatgtgcgcg cccgcgttcc 5940

tgctggcgct gggcctgttt ctggcgctgg acttcccgct gttccgtcag cagcttttcg 6000

cccacggcct tgatgatcgc ggcggccttg gcctgcatat cccgattcaa cggccccagg 6060

gcgtccagaa cgggcttcag gcgctcccga aggt 6094